(19) Europäisches Patentamt / European Patent Office / Office européen des brevets

(11) **EP 2 344 146 B1**

(12) **FASCICULE DE BREVET EUROPEEN**

(45) Date de publication et mention
de la délivrance du brevet:
**17.04.2013 Bulletin 2013/16**

(21) Numéro de dépôt: **09782881.8**

(22) Date de dépôt: **10.09.2009**

(51) Int Cl.:
*A61K 31/5513* (2006.01)   *A61K 31/165* (2006.01)
*A61K 31/196* (2006.01)   *A61K 31/4525* (2006.01)
*A61K 31/551* (2006.01)   *A61K 45/06* (2006.01)
*A61P 25/00* (2006.01)

(86) Numéro de dépôt international:
**PCT/EP2009/061765**

(87) Numéro de publication internationale:
**WO 2010/029131 (18.03.2010 Gazette 2010/11)**

(54) **AGENTS ANTI-CONNEXINES POUR LEUR UTILISATION EN TANT QU'AGENTS MODULATEURS DE L'EFFET THÉRAPEUTIQUE DE MOLÉCULES PSYCHOTROPES**

ANTI-CONNEXIN-MITTELN ZUR ANWENDUNG ZUR MODULIERUNG DER THERAPEUTISCHEN WIRKUNG VON PSYCHOTROPEN ARZNEIMITTELN

ANTI-CONNEXINE AGENTS FOR USE TO MODULATE THE THERAPEUTIC EFFECT OF PSYCHOTROPIC MOLECULES

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **10.09.2008 FR 0856090**

(43) Date de publication de la demande:
**20.07.2011 Bulletin 2011/29**

(73) Titulaires:
• **Commissariat à l'Énergie Atomique et aux Énergies Alternatives**
**75015 Paris (FR)**
• **Bio Modeling Systems Ou Bmsystems**
**75015 Paris (FR)**

(72) Inventeurs:
• **MOUTHON, Franck**
**F-75014 Paris (FR)**
• **CHARVERIAT, Mathieu**
**F-92130 Issy Les Moulineaux (FR)**
• **DESLYS, Jean-Philippe**
**F-78150 Le Chesnay (FR)**
• **IRIS, François**
**F-92370 Chaville (FR)**

(74) Mandataire: **Regimbeau**
**20, rue de Chazelles**
**75847 Paris Cedex 17 (FR)**

(56) Documents cités:
• **MEDA P.: "connexines, canaux jonctionnels et communications cellulaires" MEDECINE/ SCIENCES, vol. 12, no. 8-9, 1996, pages 909-920, XP002525031 cité dans la demande**
• **SALAMEH ET AL: "Pharmacology of Gap junctions. New pharmacological targets for treatment of arrhythmia, seizure and cancer?" BIOCHIMICA ET BIOPHYSICA ACTA. BIOMEMBRANES, AMSTERDAM, NL, vol. 1719, no. 1-2, 20 décembre 2005 (2005-12-20), pages 36-58, XP005211716 ISSN: 0005-2736**
• **FATEMI S H ET AL: "Chronic psychotropic drug treatment causes differential expression of connexin 43 and GFAP in frontal cortex of rats" SCHIZOPHRENIA RESEARCH, ELSEVIER, vol. 104, no. 1-3, 1 septembre 2008 (2008-09-01), pages 127-134, XP024523139 ISSN: 0920-9964 [extrait le 2008-06-27]**

## Description

La présente invention concerne l'amélioration des traitements thérapeutiques neurologiques et neuropsychiques utilisant des molécules psychotropes. Plus particulièrement, la présente invention permet la modulation et/ou la potentialisation des effets des drogues psychotropes par certaines molécules, appelées ici agents anti-connexines.

Le développement et l'utilisation de molécules à visée thérapeutique ayant pour cible le système nerveux central représentent un domaine en pleine expansion. Ce développement est toutefois confronté à de nombreuses limitations :

- les effets secondaires peuvent apparaître dès la dose thérapeutique et diminuent donc les bénéfices thérapeutiques.
- la résistance au traitement, l'intolérance au traitement, l'échappement thérapeutique et les phénomènes d'addictions sont autant de réactions individuelles aux médicaments particulièrement limitatrices dans le développement et l'utilisation en clinique de molécules psychotropes.
- un ressenti des effets thérapeutiques en décalage par rapport au moment de l'administration, phénomène qui est parfaitement documenté dans le cadre de traitements antidépresseurs, constitue une source d'incertitude considérable pour adapter la posologie.

Par ailleurs, parmi les molécules candidates au traitement de pathologies du système nerveux central, seules 8 % sont validées lors des essais cliniques et sont ensuite commercialisées. Lors des essais en phase préclinique, 20 % des molécules sont rejetées en raison de résultats toxicologiques non compatibles avec un traitement chez l'homme (Kola I, Nat Rev Drug Discov. 2004 Aug;3(8):711-5). Ces molécules pourraient néanmoins présenter un effet thérapeutique si leur action pouvait être potentialisée par une ou plusieurs autres molécules.

Dans ce contexte, il est donc important de trouver un moyen de i) contrôler au mieux les limitations thérapeutiques et les effets secondaires et ii) améliorer l'efficacité de molécules destinées à traiter les pathologies du système nerveux central.

Différentes études se sont déjà intéressées à la modulation des effets secondaires des traitements. Les quelques tentatives décrites ont, de façon massive, porté sur des molécules ou des peptides ciblant des récepteurs :

- PKR1 dans les phénomènes de nociception (Negri L, The Journal of Neuroscience, 2006 Jun 21;26(25):6716-27)),
- un antagoniste non-stéroïdien des récepteurs glucocorticoïdes dans les cas d'hypoglycémie extrême suite au traitement chronique par insuline (Kale AY 2006, Brain Research Bulletin 2006, jul 15 ; 135 (1-2) :1-6)
- la trimegestrone, un agoniste des récepteurs progestérone qui contrecarre en partie les effets indésirables des traitements oestradiole sans compromettre leur efficacité dans le traitement de l'ostéoporose (Winneker RC, Steroids. 2003 Nov;68(10-13):915-20),
- Le diazépam, un agoniste des récepteurs GABAA, pour atténuer les effets addictifs des opiacés (Tejwani GA, Brain Res. 1998 Jun 29;797(2):305-12).

En définitive, au moment de l'invention, aucune approche visant à résoudre les problèmes liés à l'utilisation de drogues psychotropes n'avait envisagé d'intervenir sur un système global de régulation, mais plutôt sur certains récepteurs particuliers.

## Description des figures

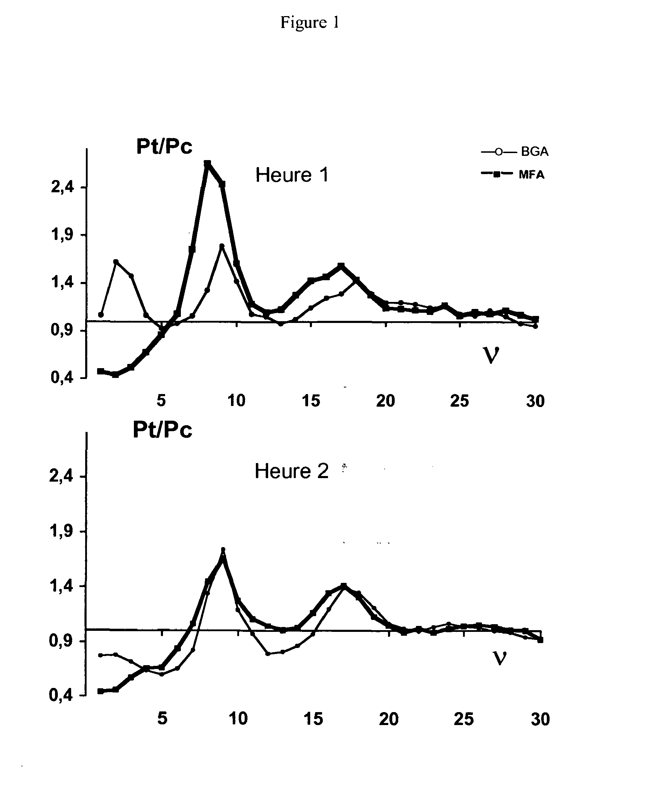

La figure 1 représente l'analyse spectrale de l'activité électrique du cortex préfrontal par EEG quantitatif de l'effet de l'injection sous méningée de l'acide Méclofénamique (MFA, en carrés noirs) ou de l'acide 18-β-Glycyrrhétinique (Beta GA, en ronds blancs). L'abscisse représente les fréquences analysées et l'ordonnée représente les puissances relatives issues de l'analyse FFT. L'analyse spectrale est représentée en moyenne pour la première heure et pour la seconde heure d'enregistrement.

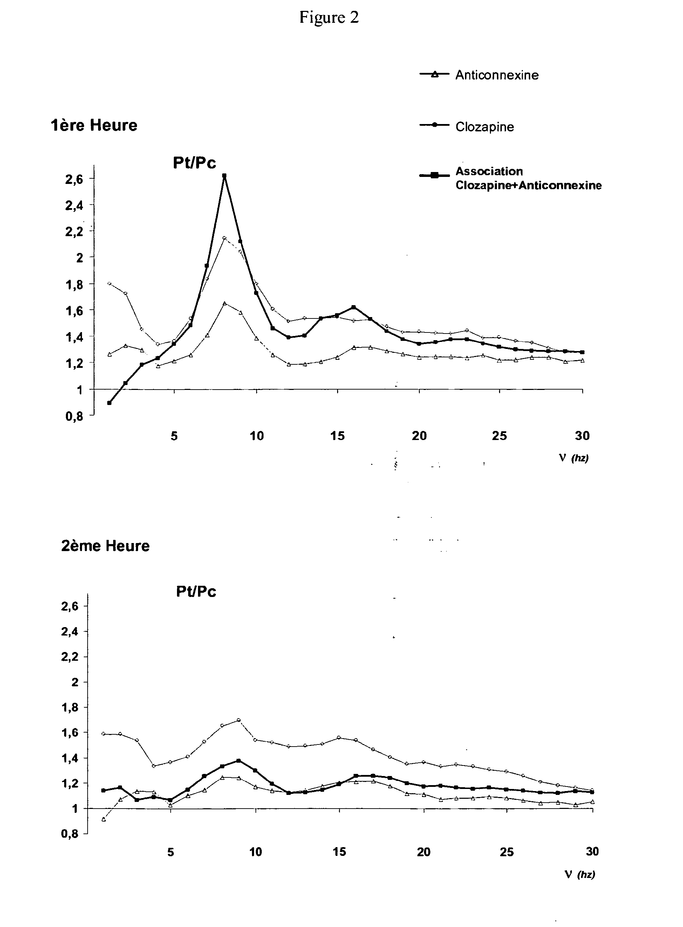

La figure 2 représente l'analyse spectrale de l'activité électrique du cortex préfrontal par EEG quantitatif de l'effet de la Clozapine seule (0,2mg/kg par voie intra péritonéale), de l'inhibiteur de connexine seul (Acide Méclofénamique, 80 ng/kg par injection sous méningée), ou de l'association [Clozapine et inhibiteur de connexine]. L'abscisse représente les fréquences analysées et l'ordonnée représente les puissances relatives issues de l'analyse par transformée de Fourier (FFT). L'analyse spectrale est représentée en moyenne pour 6 animaux pour la première heure (en haut) et la seconde heure (en bas).

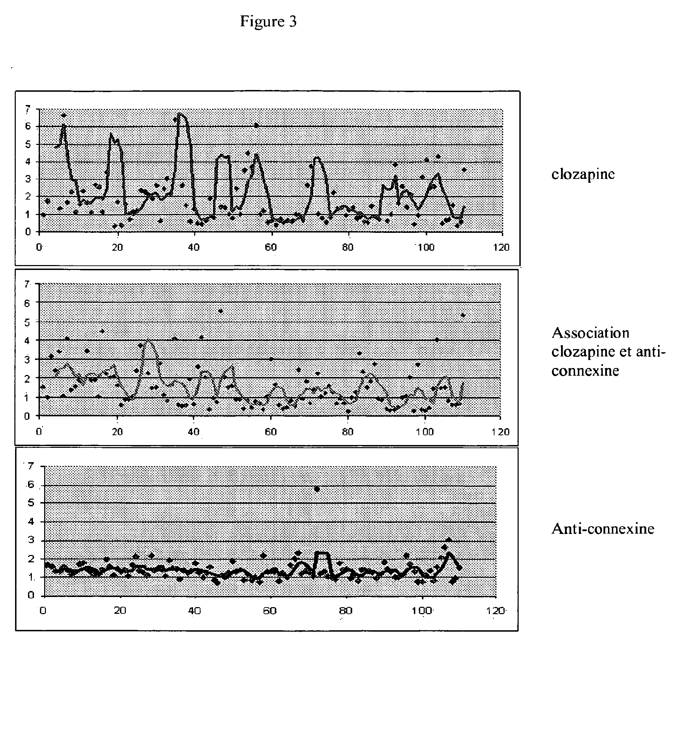

La figure 3 représente l'analyse de l'évolution minute par minute de l'activité électrique du cortex préfrontal de fréquence moyenne 8 Hz par EEG quantitatif de l'effet de la Clozapine seule (0,2mg/kg par voie intra péritonéale), de l'inhibiteur de connexine seule (Acide Méclofénamique, 80 ng/kg par injection sous méningée), ou de l'association [Clozapine et inhibiteur de connexine]. L'abscisse représente le temps en minute et l'ordonnée représente les puissances relatives issues de l'analyse FFT.

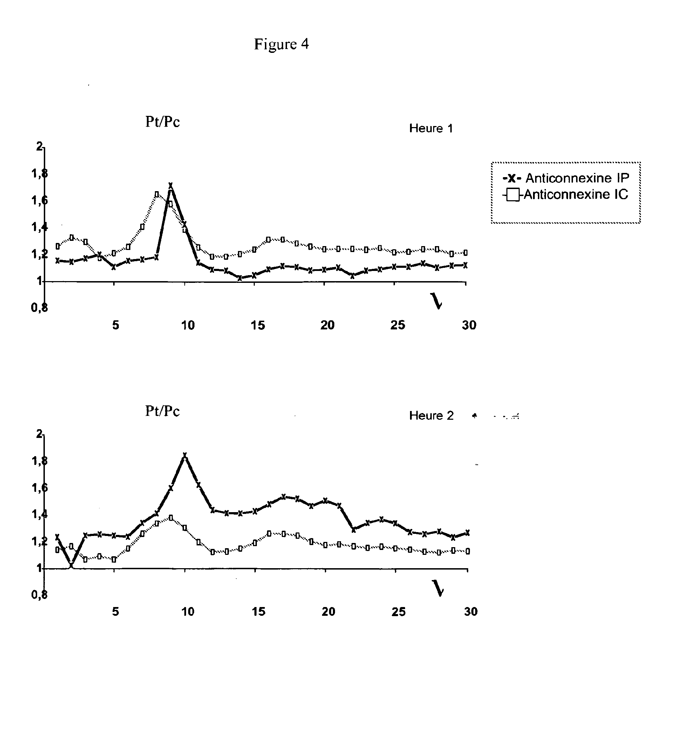

La figure 4 représente l'analyse spectrale de l'activité électrique du cortex préfrontal par EEG quantitatif de l'effet de l'inhibiteur de connexine par injection sous-méningée (Acide Méclofénamique, 80 ng/kg) ou par injection intra-peritoneale (Acide Méclofénamique, 2,45 mg/kg). L'abscisse représente les fréquences analysées et l'ordonnée représente les puissances relatives issues de l'analyse FFT. L'analyse spectrale est représentée en moyenne pour 6 animaux pour la première heure et pour la seconde heure d'enregistrement.

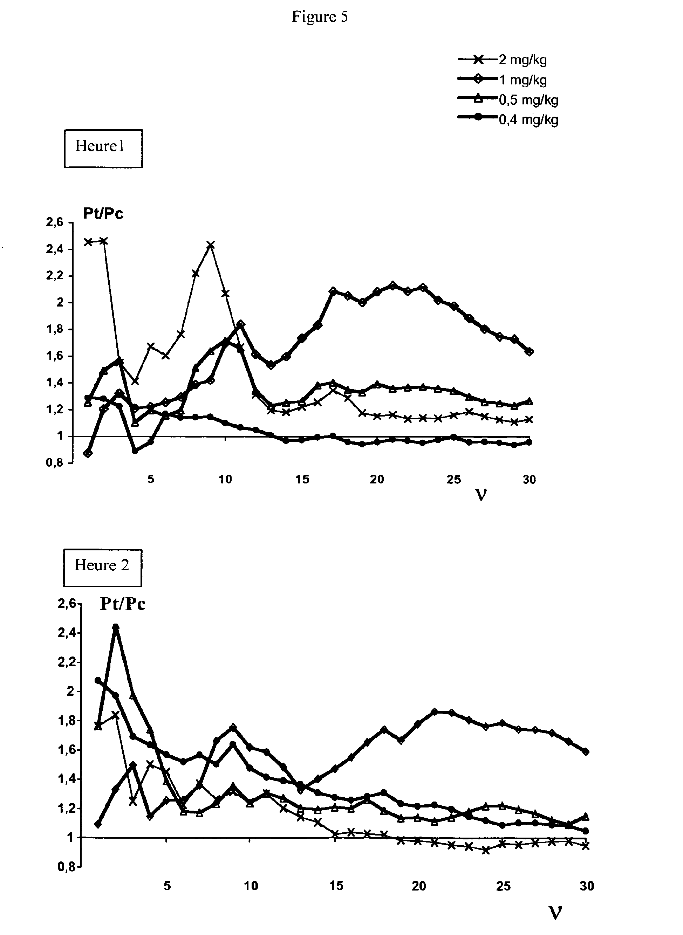

La figure 5 représente l'analyse spectrale de l'activité électrique du cortex préfrontal par EEG quantitatif de l'effet de différentes doses de l'inhibiteur de connexine par injection intra-péritonéale (Acide Méclofénamique, 2mg/kg, 1mg/kg, 0,5mg/kg, 0,4mg/kg). L'abscisse représente les fréquences analysées et l'ordonnée représente les puissances relatives issues de l'analyse FFT. L'analyse spectrale est représentée en moyenne pour la première heure et pour la seconde heure d'enregistrement.

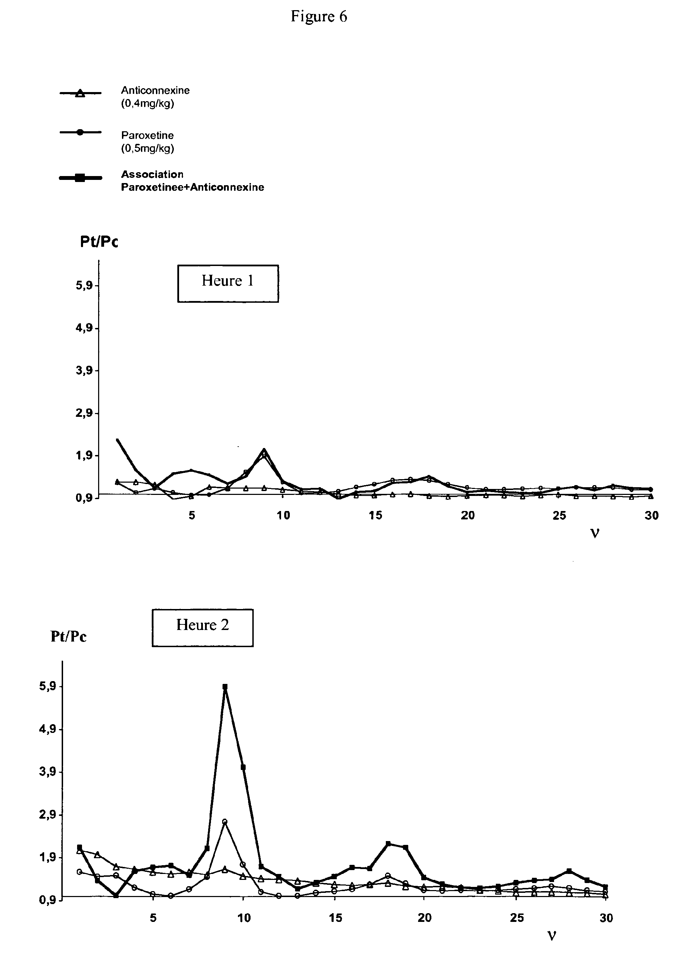

La figure 6 représente l'analyse spectrale de l'activité électrique du cortex préfrontal par EEG quantitatif de l'effet de la Paroxetine (0,5mg/kg par voie intrapéritonéale) seule, de l'inhibiteur de connexine (Acide Méclofénamique 0,4mg/kg par injection intrapéritonéale) seul, ou de l'association Paroxetine et inhibiteur de connexine. L'abscisse représente les fréquences analysées et l'ordonnée représente les puissances relatives issues de l'analyse FFT. L'analyse spectrale est représentée en moyenne pour les 6 animaux pour la première heure et pour la seconde heure.

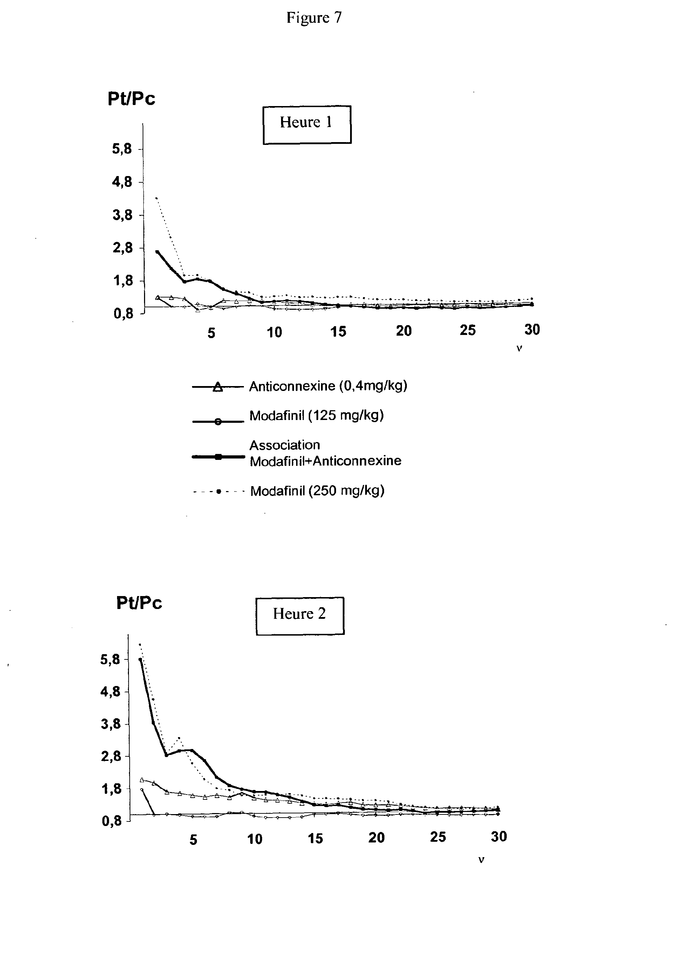

La figure 7 représente l'analyse spectrale de l'activité électrique du cortex préfrontal par EEG quantitatif de l'effet du Modafinil (125mg/kg et 250mg/kg par voie intrapéritonéale) seul, de l'inhibiteur de connexine (Acide Méclofénamique 0,4mg/kg par injection intrapéritonéale) seul, ou de l'association Modafinil (125mg/kg par voie intrapéritonéale) et inhibiteur de connexine. L'abscisse représente les fréquences analysées et l'ordonnée représente les puissances relatives issues de l'analyse FFT. L'analyse spectrale est représentée en moyenne pour les 6 animaux pour la première heure et pour la seconde heure.

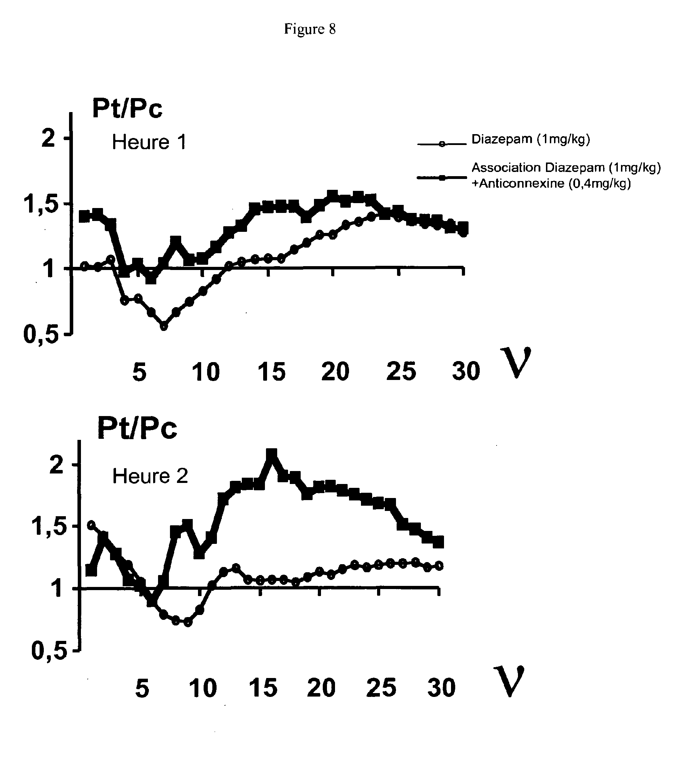

La figure 8 représente l'analyse spectrale de l'activité électrique du cortex préfrontal par EEG quantitatif de l'effet du Diazepam (1mg/kg par voie intrapéritonéale) seule, ou de l'association du Diazepam (1mg/kg par voie intrapéritonéale) avec l'inhibiteur de connexine (Acide Méclofénamique, 0,4mg/kg par voie intrapéritonéale). L'abscisse représente les fréquences analysées et l'ordonnée représente les puissances relatives issues de l'analyse FFT. L'analyse spectrale est représentée en moyenne pour les 6 animaux pour la première heure et pour la seconde heure.

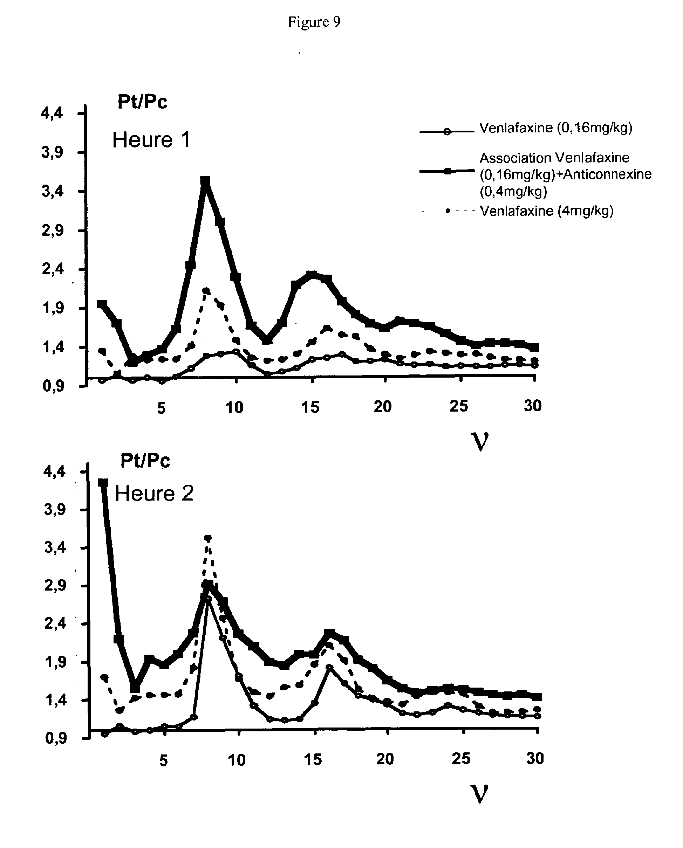

La figure 9 représente l'analyse spectrale de l'activité électrique du cortex préfrontal par EEG quantitatif de l'effet de la Venlafaxine (0,16mg/kg et 4mg/kg par voie intrapéritonéale) seule, ou de l'association de la Venlafaxine (0,16mg/kg par voie intrapéritonéale) avec l'inhibiteur de connexine (Acide Méclofénamique, 0,4mg/kg par voie intrapéritonéale). L'abscisse représente les fréquences analysées et l'ordonnée représente les puissances relatives issues de l'analyse FFT. L'analyse spectrale est représentée en moyenne pour les 6 animaux pour la première heure et pour la seconde heure.

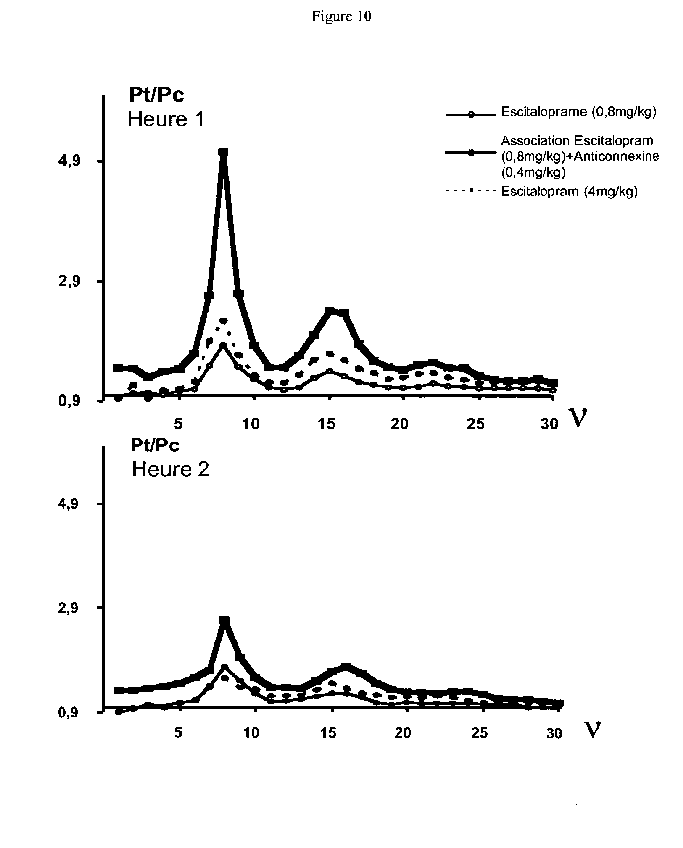

La figure 10 représente l'analyse spectrale de l'activité électrique du cortex préfrontal par EEG quantitatif de l'effet de l'Escitalopram (0,8mg/kg et 4mg/kg par voie intrapéritonéale) seule, ou de l'association de l'Escitalopram (0,8mg/kg par voie intrapéritonéale) avec l'inhibiteur de connexine (Acide Méclofénamique 0,4mg/kg par voie intrapéritonéale). L'abscisse représente les fréquences analysées et l'ordonnée représente les puissances relatives issues de l'analyse FFT. L'analyse spectrale est représentée en moyenne pour les 6 animaux pour la première heure et pour la seconde heure.

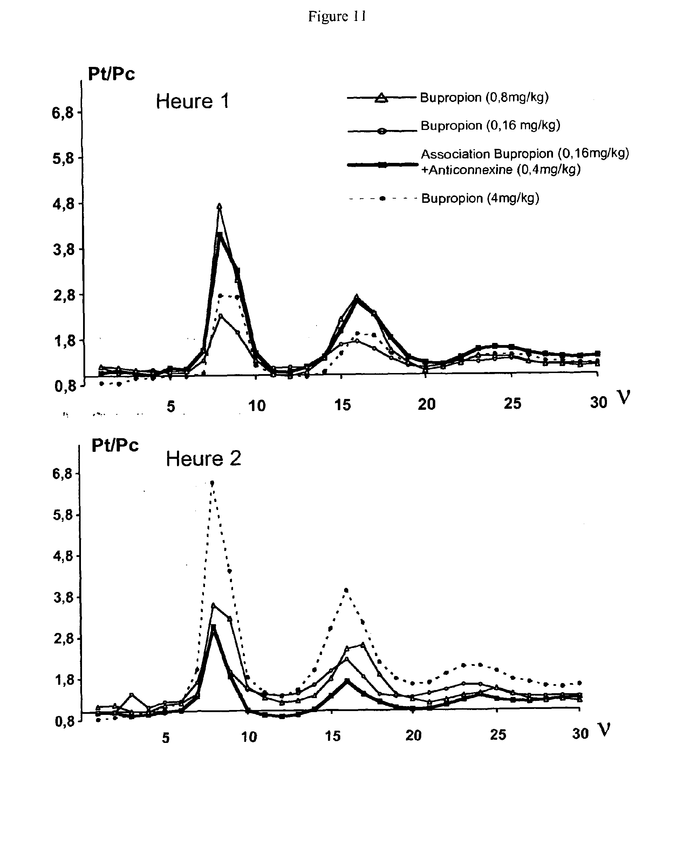

La figure 11 représente l'analyse spectrale de l'activité électrique du cortex préfrontal par EEG quantitatif de l'effet du Bupropion (0,16mg/kg, 0,8mg/kg et 4mg/kg par voie intrapéritonéale) seule, ou de l'association du Bupropion (0,16mg/kg par voie intrapéritonéale) avec l'inhibiteur de connexine (Acide Méclofénamique, 0,4mg/kg par voie intrapéritonéale). L'abscisse représente les fréquences analysées et l'ordonnée représente les puissances relatives issues de l'analyse FFT. L'analyse spectrale est représentée en moyenne pour les 6 animaux pour la première heure et pour la seconde heure.

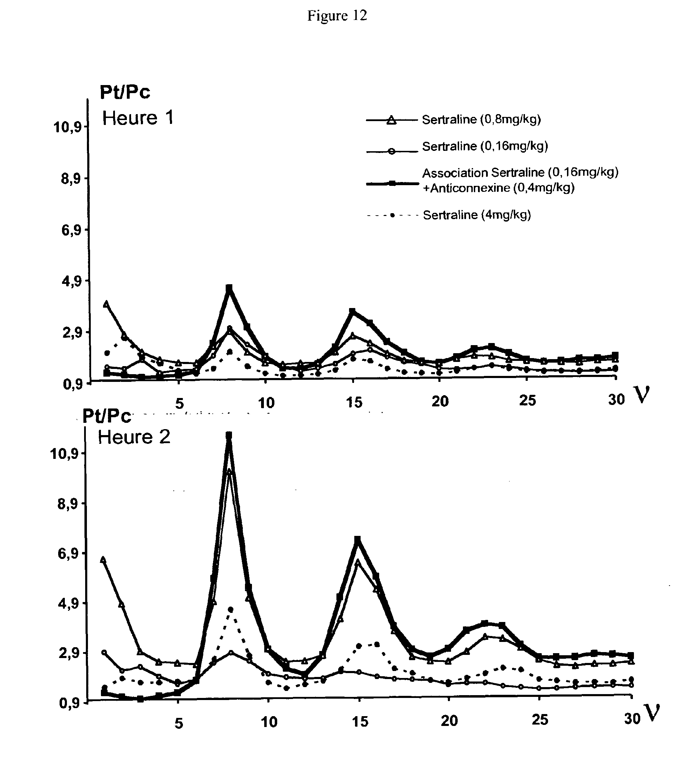

La figure 12 représente l'analyse spectrale de l'activité électrique du cortex préfrontal par EEG quantitatif de l'effet de la Sertraline (0,16mg/kg, 0,8mg/kg et 4mg/kg par voie intrapéritonéale) seule, ou de l'association de la sertraline (0,16mg/kg par voie intrapéritonéale) avec l'inhibiteur de connexine (Acide Méclofénamique, 0,4mg/kg par voie intrapéritonéale). L'abscisse représente les fréquences analysées et l'ordonnée représente les puissances relatives issues de l'analyse FFT. L'analyse spectrale est représentée en moyenne pour les 6 animaux pour la première heure et pour la seconde heure.

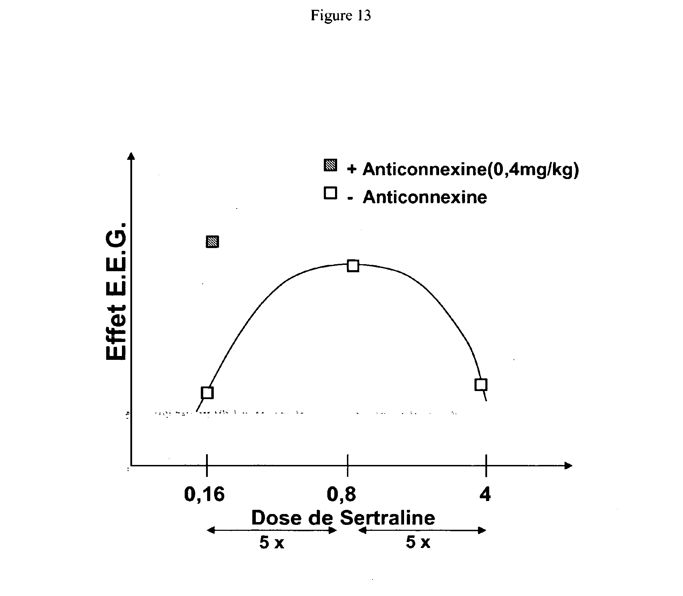

La figure 13 est une représentation schématique de l'effet EEG en « U » inversé de la Sertraline seule selon la dose administrée sur les deux heures d'enregistrement (0,16mg/kg, 0,8mg/kg et 4mg/kg par voie intrapéritonéale), ou de l'association sertraline (0,16mg/kg par voie intrapéritonéale) avec l'inhibiteur de connexine (Acide Méclofénamique, 0,4mg/kg par voie intrapéritonéale).

Description de l'invention

La communication intercellulaire est importante pour la maintenance de l'homéostatie tissulaire et organique. Pour établir cette communication, des jonctions lacunaires relient le cytoplasme des cellules, permettant l'échange d'ions ($Ca^+$ et K+), de seconds messagers (AMPc, GMPc, IP3), de quelques métabolites de petite taille (glucose), et assurant un couplage électrique et métabolique entre les cellules. Les jonctions lacunaires sont des jonctions de perméabilité sélective, formées de canaux protéiques enchâssés dans la membrane plasmique, et constitués d'hexamères de connexines (Meda P, Médecine/Sciences 1996; 12 :909-920).

Les connexines sont des protéines intégrales de la membrane plasmique, qui sont synthétisées pratiquement par chaque type cellulaire, quelle que soit la position d'un organisme multicellulaire dans la phylogénèse du monde animal. Chez les vertébrés, les rares cellules ne produisant pas de connexines sont les cellules musculaires striées adultes, les spermatozoïdes et les cellules circulantes du sang. Contrairement à de nombreuses protéines de membrane, les connexines ont une durée de demi-vie courte (entre 3 et six heures), ne sont pas glycosylées et n'ont pas d'activité enzymatique. A ce jour, au moins treize connexines distinctes ont été identifiées chez les mammifères ; correspondant, chez l'homme, à 21 isoformes. En pratique, différents types de connexine peuvent être présents dans plusieurs tissus, et la plupart des cellules synthétisent plusieurs connexines (Meda P, Médecine/Sciences 1996; 12 :909-920). Avant d'atteindre la membrane cellulaire, les connexines s'assemblent par groupes de six molécules pour former des structures tubulaires creuses appelées connexons, qui rejoignent la membrane plasmique grâce à des vésicules golgiennes. Lorsqu'un contact cellulaire est établi, les connexons d'une cellule s'alignent bout à bout avec ceux de la cellule voisine, établissant un canal hydrophile continu long de 10 nm environ. Ce canal jonctionnel met en communication directe le cytoplasme des deux cellules en contact, par delà l'espace intercellulaire.

Ainsi la formation de duplex d'hexamères entre deux membranes plasmiques adjacentes constitue la jonction lacunaire. Le nombre de gènes codant pour différentes isoformes de connexines est au nombre de 21 chez l'homme et il est décrit différentes combinaisons de monomères de connexines rentrant dans la composition des jonctions lacunaires. La moitié des connexines identifiées est exprimée dans le cerveau, et plus particulièrement la connexine 36 (Cx36), qui semble majoritairement exprimée par les neurones, au sein d'une interface définie comme la synapse électrique. Les souris déficientes pour la Connexine 36 ne présentent aucun couplage inter neurones, ce qui contribue à affirmer le rôle prépondérant de ces connexines particulières dans au moins un type de synapse électrique (Wellershaus K, Exp Cell Res. 2008).

Les synapses électriques ne transmettent pas l'information plus rapidement que les synapses chimiques, mais présentent une caractéristique particulière, celle de la réciprocité : ni inhibitrices, ni excitatrices, elles synchronisent localement l'état de plusieurs cellules et ce de façon bilatérale. Les jonctions lacunaires agissant comme des filtres passe-bas, ce processus de normalisation de l'état des cellules est d'autant plus efficace qu'il implique des changements à plus ou moins long terme. Les couplages électriques entre neurones, en vertu de leurs caractéristiques, joueraient un rôle dans les phénomènes d'oscillations et de décharges rythmiques dans le néocortex et l'hippocampe.

En définitive, les connexines sont présentes de façon ubiquitaire dans les différentes structures du cerveau et sont impliquées localement dans des phénomènes d'oscillations des activités électriques.

Or, les présents inventeurs ont récemment démontré que les connexines jouent en plus un rôle important dans la régulation globale de l'activité électrique du cerveau. De façon surprenante, lorsqu'elles sont présentes de façon physiologique, ces protéines ont en effet un rôle désynchronisateur global de l'activité électrique du SNC, amortissant et neutralisant potentiellement les phénomènes d'emballement. A l'inverse, l'inhibition de ces molécules au moyen d'agents dits « anti-connexines » permet de synchroniser, et donc d'augmenter l'activité électrophysiologique mesurée.

Comme il est connu que l'augmentation de l'activité électrique du SNC, mesurée sur un électroencéphalogramme (EEG), reflète, dans certaines conditions, les bénéfices thérapeutiques d'une drogue psychotrope (Galderisi S, Methods Find Exp Clin Pharmacol, 2002, 24, 85-89), les présents inventeurs ont eu l'idée de tester l'effet de l'inhibition des connexines sur l'effet thérapeutique lié à l'administration de drogues psychotropes.

Leurs nombreux résultats prouvent sans ambiguïté que la modulation de l'activité électrique cérébrale liée à l'administration d'agents anti-connexines, permet d'obtenir un bénéfice de dose de drogues psychotropes auxquels ils sont associés et de disposer d'une méthode d'évaluation de ce bénéfice de dose basée sur cette modulation. L'association d'un agent anti-connexine avec une molécule psychotrope, par exemple un antidépresseur, permet donc i) d'augmenter la spécificité d'action, et les effets thérapeutiques de la molécule psychotrope, et ii) de diminuer les doses actives et ainsi réduire les effets indirects de ces molécules psychotropes (effets indésirables, échappement, résistance).

Selon un premier aspect, la présente invention propose une méthode d'évaluation de la dose équivalente efficace de drogue psychotrope qui pourra être associée à l'agent bloquant les connexines lors du traitement de patient souffrant de troubles psychiatriques et/ou neurodégénératifs. Cette association a pour but d'obtenir le même effet thérapeutique que celui de la drogue psychotrope seule administrée à une dose plus forte, mais avec moins d'effets secondaires.

Dans le cadre de l'invention, on appelle « dose équivalente efficace » de drogue psychotrope la dose de drogue psychotrope qui, lorsqu'elle est administrée en combinaison avec l'agent bloquant les connexines, induit un effet physiologique ou une signature pharmacologique similaire ou identique à celui ou celle de la drogue psychotrope seule administrée à la dose pharmacologique active.

D'autre part, on désigne par « dose active pharmacologique » de drogue psychotrope la dose de drogue psychotrope classiquement administrée aux animaux de laboratoire comme le rat, la souris, le lapin, etc. De telles doses sont fournies par exemple dans Animal models in psychopharmacology. Olivier B, Slangen J, Mos J, eds. Birkhauser Verlag, Basel; 1991. Dans le cas où cette dose n'est pas connue, il est possible de déterminer la dose active pharmacologique de drogue psychotrope par transposition chez l'animal des doses actives pharmacologiques classiquement prescrites chez l'homme et qui peuvent être consultées dans « Médicaments psychotropes: consommation et pratiques de prescription en France métropolitaine. I. Données nationales, 2000 ». Lecadet J, Vidal P, Baris B etal.; Revue Médicale de l'Assurance Maladie volume 34 n° 2 / avril-juin 2003 » Il est également possible de déterminer cette dose active pharmacologique par des tests expérimentaux, la dose active pharmacologique étant la dose maximale de drogue psychotrope que l'on peut administrer à un animal sans que les effets secondaires deviennent prépondérants par rapport à l'effet thérapeutique. Dans ce cas, la dose pharmacologique active peut être déterminée de proche en proche en administrant plusieurs doses de drogue psychotrope à des doses croissantes, et en mesurant à chaque fois l'effet exercé par ladite drogue.

Dans le cadre de la présente invention, un agent « bloquant les connexines » est une molécule chimique, une protéine, un fragment de protéine, ou un acide nucléique (RNAi) capable d'inhiber l'activité fonctionnelle des connexines, de façon directe et/ou indirecte, et plus généralement tout type de jonctions intercellulaires, et/ou capable d'inhiber fonctionnellement, de façon directe et/ou indirecte, toute activité cellulaire faisant intervenir une protéine de type connexine. Un tel agent peut également être qualifié de « molécule anticonnexine».

La méthode d'évaluation de la dose équivalente efficace de drogue psychotrope est basée sur la quantification de la potentialisation de l'effet de la drogue psychotrope par l'agent bloquant les connexines. Cette évaluation est réalisée en plusieurs étapes : une étape de caractérisation des effets propres du psychotrope et une étape de mesure du bénéfice de dose du produit de combinaison. La première étape consiste à déterminer et quantifier les effets de la drogue psychotrope seule à différentes doses (dose pharmacologique active et doses décroissantes à partir de la dose pharmacologique active), et ainsi de disposer de signatures propres à la molécule psychotrope - ou signatures étalons - aux différentes doses. La seconde étape consiste à déterminer la dose de drogue psychotrope qui, administrée en combinaison avec l'agent bloquant les connexines, a la même signature pharmacologique que la drogue psychotrope seule.

De manière avantageuse, la dose d'agent bloquant les connexines utilisée en association avec la drogue psychotrope est déterminée au préalable par des tests expérimentaux. Cette dose correspond en effet à la dose maximale d'agent bloquant les connexines qui peut être administrée sans exercer d'effet pharmacologique significatif propre. Cette dose peut être ajustée lors de la mise en oeuvre de la méthode selon l'invention, pour optimiser l'effet de l'association.

La méthode d'évaluation de la dose équivalente efficace de drogue psychotrope selon l'invention comprend les étapes suivantes:

a) disposer d'autant de lots d'animaux que de conditions expérimentales à tester,

b) administrer à un premier lot d'animaux une dose active pharmacologique de ladite drogue psychotrope, et à des lots successifs d'animaux des doses décroissantes à partir de ladite dose active pharmacologique active,

c) caractériser, chez les animaux desdits lots, l'effet exercé par ladite drogue psychotrope aux différentes doses administrées,

d) administrer, à de nouveaux lots d'animaux, ledit produit de combinaison contenant ladite drogue psychotrope aux doses utilisées en b), et ledit agent bloquant les connexines,

e) caractériser, chez les animaux desdits lots, l'effet exercé par chacun desdits produits de combinaison administrés en d),

f) déterminer la dose équivalente efficace de drogue psychotrope, i.e. la dose de drogue psychotrope qui, lorsqu'elle est administrée en combinaison avec l'agent bloquant les connexines, exerce le même effet que celui de la drogue psychotrope seule administrée à ladite dose active pharmacologique.

Cette méthode d'évaluation de la dose équivalente efficace de drogue psychotrope permet en outre d'évaluer le bénéfice de dose entre, d'une part, un produit de combinaison associant ladite drogue psychotrope à la dose équivalente efficace et un agent bloquant les connexines, et, d'autre part, la drogue psychotrope administrée à la dose pharmacologiquement active.

Le terme « bénéfice de dose » désigne, dans la présente demande, le rapport entre la dose pharmacologique de la drogue psychotrope seule et la dose équivalente efficace de la drogue psychotrope. En d'autres termes, le bénéfice de dose explicite de combien la dose pharmacologique de drogue psychotrope prescrite peut être diminuée en vue de son association avec l'agent anti-connexine. Cette diminution de la dose de drogue psychotrope administrée (en association avec les molécules anticonnexines) n'aura pas de conséquence en termes d'efficacité du traitement, mais entrainera une diminution des effets secondaires.

Ainsi, le bénéfice de dose défini plus haut peut s'écrire :

$$\mathrm{B}D = \frac{\text{Dose } active \text{ pharmacologique de la drogue psychotrope}}{\text{Dose équivalente efficace de la drogue psychotrope}}$$

Il est entendu que, pour réaliser la méthode d'évaluation selon l'invention, lesdits lots d'animaux sont de la même espèce. Par ailleurs, chaque lot d'animaux reçoit exclusivement soit une dose de drogue psychotrope seule, soit un produit de combinaison contenant une certaine dose de drogue psychotrope et un agent anti-connexine, de sorte à ne mesurer, chez les animaux du même lot, que l'effet de ladite drogue ou dudit produit de combinaison seuls. De manière préférée, lesdits lots d'animaux ont le même âge et sont du même sexe. Ces animaux sont de préférence des animaux de laboratoire, par exemple rats, souris, lapins, etc.

Il est entendu que le nombre de doses à tester est déterminé en fonction de l'effet pharmacologique de la drogue psychotrope ou du produit de combinaison associant la drogue psychotrope et l'agent bloquant les connexines ; lorsque l'effet pharmacologique exercé par une certaine dose est nul ou dérisoire, il n'est plus nécessaire d'utiliser des doses plus faibles.

L'administration de la drogue ou du produit de combinaison peut être effectuée par voie intracérébrale, mais est de préférence effectuée par voie intrapéritonéale.

De manière avantageuse, l'effet exercé par la drogue psychotrope et/ou le produit de combinaison peut être déterminé par différents types d'analyse, en particulier une analyse électrophysiologique ou comportementale ou de marqueurs sanguins ou de marqueurs LCR, ou par imagerie médicale. De préférence, cet effet est déterminé par référence à une réponse électrophysiologique consécutive à une stimulation donnée, en particulier en référence à l'activité électroencéphalographique (EEG) de l'animal.

De manière avantageuse, cette méthode d'évaluation permet de définir le schéma temporel optimal pour l'administration des deux éléments du produit de combinaison (administration simultanée, séparée ou étalée dans le temps).

Cette méthode d'évaluation permet également d'opérer une sélection en ce qui concerne la nature de l'agent bloquant les connexines, de la drogue psychotrope, ainsi que des doses utilisées.

Selon un second aspect, la présente invention concerne un nouveau produit de combinaison contenant au moins un agent bloquant les connexines et une drogue psychotrope, et son utilisation chez des patients souffrant de troubles psychiatriques et/ou neurodégénératifs.

Différentes molécules sont connues pour bloquer les jonctions lacunaires via les connexines.

Parmi elles, la famille des fénamates comprend les composés suivants : l'acide méclofénamique, l'acide flufénamique, l'acide niflumique, l'acide tolfénamique. Ces composés ont tous une activité non-stéroïdienne anti-inflammatoire, mais cette activité n'est pas responsable de leur capacité à bloquer les jonctions lacunaires. Il a en effet été suggéré que les fenamates établissaient plutôt une interaction directe avec les connexines ou avec les interfaces membranaires de protéines qui pourraient influer sur la conformation des connexines et par conséquent sur leur rôle fonctionnel (Harks EG, The Journal of Pharmacology and Experimental Therapeutics 2001 Sep, 298(3) : 1 033-41)

L'acide 2-[(2,6-di-chloro-3-phényl)amino] benzoïque, plus communément connu sous le nom d'acide méclofénamique (MFA), est un agent anti-inflammatoire non stéroïdien et un analgésique périphérique de la classe des fénamates, inhibiteur des prostaglandines, décrit parmi les bloquants hydrosolubles comme étant le plus efficace pour bloquer les jonctions lacunaires de façon réversible. De plus, l'acide méclofénamique n'est pas spécifique d'un type de connexine et est donc efficace pour bloquer un grand nombre de connexines cérébrales (Pan F, Vis Neurosciences 2007, Jul-Aug; 24(4):609-18)

Les dérivés d'acide glycyrrhétinique désignent les acides 18-β-glycyrrhétinique (BGA) également connu sous le nom de « enoxolone », 18-α-glycyrrhétinique et la carbenoxolone, , qui sont des saponines triterpinoïdes connues pour inhiber l'enzyme 11-hydroxystéroïde dehydrogénase. Par ailleurs, ces composés sont capables d'inhiber les jonctions lacunaires de façon très efficace (Pan F, Vis Neurosciences 2007, Jul-Aug; 24(4):609-18).

La méfloquine (LARIAM), de la famille des quinines, a également un fort pouvoir antagoniste sur les jonctions lacunaires (Srinivas M, PNAS 2001, 98 :10942-10947 ; Pan F, Vis Neurosciences 2007, Jul-Aug; 24(4):609-18).

Certains agents anesthésiants, tels l'halothane et l'isoflurane, ont un effet rapide et réversible de blocage des jonctions lacunaires (Burt JM, et al, Circ Research. 1989 ; 65 :829-37).

Par ailleurs, l'oléamide (cis-9-octadecenamide), le premier amide de l'acide oléique, a également une action inhibitrice sur les molécules connexines 43 et 32 (Guan X. et al, J. Cell Biol 1997; 139 :1785-92).

De plus, les cyclodextrines (l'α-cyclodextrine (α-CD), la β-cyclodextrine (β-CD), et la γ-cyclodextrine (γ-CD)), qui sont des oligosaccharides cycliques naturels de α-D-glucopyranose, ont des propriétés anti-connexines avérées (Locke D. et al, J Biol Chem 2004 ; 279: 22883-92).

Enfin, le 2-aminoethyldiphényl borate (2-APB) est un composé récemment identifié comme agent bloquant les jonctions lacunaires (Bai D, J Pharmacol Exp Ther, 2006 Dec ; 319(3) :1452-8). Ce modulateur du récepteur à l'inositol 1,4,5-triphosphate cible cependant de façon assez spécifique certaines connexines, comme les connexines 26, 30, 36, 40, 45, et 50 (Bai D, J Pharmacol Exp Ther, 2006 Dec ; 319(3) :1452-8).

Parallèlement, d'autres molécules ont été récemment proposées pour bloquer le domaine extracellulaire des connexines - domaine qui est important pour le fonctionnement des jonctions lacunaires. Il s'agit notamment d'anticorps dirigés contre le domaine extracellulaire des connexines (Hofer A et al, Glia 1998 ; 24 :141-54 ; Meyer RA, J. Cell Biol. 1992 ; 119 : 179-89) ou encore des petits peptides mimant des séquences spécifiques conservées des boucles extracellulaires E1 et E2 des connexines (Dahl G. et al, Biophys J, 1994 ; 67 :1816-22); notamment, les peptides correspondants aux séquences extracellulaires incluant les motifs conservés QPG et SHVR de E1 (Gap26) et le motif conservé SRPTEK de E2 (Gap 27) des connexines sont les plus efficaces pour bloquer les jonctions lacunaires (Chaytor AT et al, J. Physiol 1997 ; 503 :99-110).

Dans le cadre de la présente invention, les agents bloquant les connexines sont avantageusement choisis parmi: les alcools à longues chaines (par exemple heptanol et octanol), les fénamates (par exemple l'acide méclofénamique, l'acide flufénamique, l'acide niflumique, l'acide tolfénamique), les arylaminobenzoates, les aminosulfonates (par exemple la taurine), les dérivés d'acides glycyrrhétinique (par exemple l'acide 18-β-glycyrrhétinique, l'acide 18-α-glycyrrhétinique et la carbenoxolone), les oléamides (par exemple la cis-9-octadecenamide), ou encore les ions tetraalkylammonium et les polyamines (telles que la spermine et la spermidine), les dérivés de quinine (comme la méfloquine), le 2-ABP, les agents anesthésiants (halothane ou isoflurane), les cyclodextrines (l'α-cyclodextrine (α-CD), la β-cyclodextrine (β-CD), et la γ-cyclodextrine (γ-CD)), les anticorps dirigés contre le domaine extracellulaire des connexines ou encore les peptides à motifs conservés mimant ce domaine particulier (en particulier Gap26 et Gap27). Ces différentes molécules sont précisément décrites dans l'articles suivant : Srivinas M, Connexins : a guide, Humana Press 2009, chapter 8, pp 207-224 ; Srinivas M, Molecular Pharmacology 2003 jun, 63(6) :1389-97 ; Harks EG, The Journal of Pharmacology and Experimental Therapeutics 2001 Sep, 298(3) :1033-41; et Salameh A, Biochimica et Biophysica Acta 1719 (2005) 36-58.

De manière préférée, l'agent bloquant les connexines est compris dans le groupe comprenant l'acide méclofénamique, l'acide 18-β-glycyrrhétinique, le carbenoxolone, la méfloquine et le 2-APB, de manière encore plus préférée dans le groupe comprenant l'acide méclofénamique, l'acide 18-β-glycyrrhétinique et le carbenoxolone.

Ces composés sont donnés à titre d'exemple, et l'invention concerne toute molécule possédant des propriétés de blocage fonctionnel, directes ou indirectes, des connexines ou des jonctions lacunaires.

Certaines molécules ayant une fonction anti-connexine reconnue ont également été décrites pour leur effet anti-inflammatoire, anesthésique, ou encore sur l'homéostasie de la prostaglandine, et présentent donc, par eux-mêmes, des effets sur le système nerveux central. Cependant, aux doses auxquelles sont utilisées ces molécules dans l'invention (doses très faibles), les activités autres que celles anticonnexine ne participent pas à ces effets. En outre, l'utilisation de doses faibles permet une meilleure spécificité tissulaire dépendante de la composition en connexine du tissu, car le SNC est particulièrement riche en connexine.

Enfin, il convient de noter que les molécules anti-inflammatoires peuvent entraîner, indirectement, par leur action sur la Prostaglandine synthase, une modification structurelle des connexines (la régulation des niveaux d'expression de connexine ou de leur phosphorylation se font notamment via PI3K et PKA, elles-mêmes dépendantes des niveaux d'activité des Cox, NO et PG synthétases, cibles des anti-inflammatoires). Cette modification, dans le sens d'une diminution de présence des connexines au sein des jonctions, entraîne indirectement une diminution de l'activité fonctionnelle des connexines similaire à un blocage direct des connexines. Par conséquent; l'utilisation de ces molécules aboutira à l'effet recherché (blocage des connexines) et ne représente pas un obstacle à leur utilisation en association à faible dose avec des psychotropes ou psychotropes (Yao J, Morioka T & Oite T.: Kidney Int. 2000;57:1915-26.Yao J, Hiramatsu N, Zhu Y, et al. : J Am Soc Nephrol. 2005;16:58-67 ; Figueroa XF, Alviña K, Martinez AD, et al. : Microvasc Res. 2004;68:247-57Alldredge BT.: J Clin Pathol. May 12 2008 ; Lai-Cheong JE, Arita K & McGrath JA. : J Invest Dermatol. 2007;127:2713-25, et Giepmans BN.: Cardiovasc Res. 2004;62:233-45).

Par ailleurs, la formation des jonctions lacunaires fonctionnelles peut être régulée par l'intermédiaire de la phosphorylation des connexines. En effet, la phosphorylation de certains domaines protéiques des sous-unités d'hexamère conduit à une inhibition de la fonctionnalité des jonctions lacunaires, selon le site de phosphorylation, par fermeture des canaux ou par diminution de la présence à la membrane (modification du trafic et de la demi-vie des sous-unités) (Scemes E, Glia 2008 Jan 15, 56(2): 145-53 ; Postma FR, J Cell Biol 1998 Mar 9, 140(5):1199-209 ; Shaw RM, Cell 2007, February 9, 128(3):547-60; Fabrizi GM, Brain 2007 feb, 130(Pt2):394-403).

Ainsi, des molécules peuvent avoir un effet indirect de blocage des jonctions lacunaires, via les niveaux de phosphorylation des connexines. Ce sont notamment : l'acide lysophosphatidique, la thrombine et des neuropeptides, comme l'endotheline (Postma FR, J Cell Biol 1998 Mar 9, 140(5):1199-209).

Dans un mode de réalisation préféré de l'invention, l'agent bloquant les connexines a un effet indirect sur les connexines et les jonctions lacunaires, et il est choisi dans le groupe constitué par : l'acide lysophosphatidique, la thrombine et des neuropeptides, comme l'endotheline.

Dans un mode préféré de l'invention, l'agent bloquant les connexines n'est ni un agent anesthésiant, ni un agent anti-inflammatoire, ni un agent inhibiteur de la synthèse de la prostaglandine. Selon ce mode de réalisation préféré, l'agent bloquant les connexines est le 2-amino ethoxy diphényl borate (2-APB).

L'agent : bloquant les connexines peut avantageusement améliorer l'effet thérapeutique des drogues psychotropes prescrites par un médecin pour traiter un patient souffrant de l'un des troubles psychiatriques et/ou neurodégénératifs. Chez l'animal, cette amélioration peut être mesurée par la méthode d'évaluation de dose équivalente efficace qui est le premier objet de l'invention.

On entend par « psychotrope » toute substance qui agit principalement sur l'état du système nerveux central en y modifiant certains processus biochimiques et physiologiques cérébraux.

La présente invention se distingue de l'art antérieur par le fait que, dans le produit de combinaison, les bénéfices thérapeutiques apportés par l'agent bloquant les connexines ne sont pas spécifiques à l'utilisation d'une drogue psychotrope en particulier, mais s'appliquent de façon similaire à de nombreuses molécules ayant des effets psychotropes.

De manière préférée, les drogues psychotropes sont choisies parmi les psychotropes effecteurs dopaminergiques, GABAergiques, adrénergiques, acétylcholinergiques, sérotoninergiques, opioïdergiques, adénosinergiques, ionotropiques, histaminergiques, IMAO, Catéchol-O-méthyl transférase, DOPA decarboxylase, noradrénergiques.

Le terme « effecteur » désigne toute substance activant ou inhibant un ou plusieurs neurorécepteurs, il peut donc s'agir d'un agoniste ou d'un antagoniste des dits récepteurs.

Selon un mode de réalisation particulier, la drogue psychotrope est un effecteur dopaminergique tel que la loxapine, l'acepromazine, le méthylphenidate, l'amantadine, le pergolide, la lisuride, la bromocriptine, le ropinirole, l'apomorphine, l'aripiprazole, le sulpiride, l'amisulpride, le sultopride, le tiapride, le pimozide, la risperidone, l'haloperidol, le penfluridol, le zuclopenthixol ou le bupropion.

Selon un autre mode de réalisation particulier, la drogue psychotrope est un effecteur GABAergique tel que le tiagabine, le topiramate, le clorazepate, le diazepam, le clonazepam, l'oxazepam, le lorazepam, le bromazepam, le lormetazepam, le nitrazepam, le clotiazepam, l'alprozolam, l'estazolam, le triazolam, le loprazolam, l'etifoxin, le meprobamate, le zopiclone, le zolpidem, le phenobarbital, le felbamate, la vigabatrine.

Selon un autre mode de réalisation particulier, la drogue psychotrope est un effecteur adrénergique tel que la dihydroergotamine, le modafinil, l'adrafinil, la mirtazapine, l'oxetorone.

Selon un autre mode de réalisation particulier, la drogue psychotrope est un effecteur acétylcholinergique tel que la sulbutiamine, la tropatepine, le trihexyphenidyle.

Selon un autre mode de réalisation particulier, la drogue psychotrope est un effecteur sérotinergique tel que la chlorpromazine, la trimipramine, la clozapine, l'olanzapine, la cyamemazine, le flupentixol, le nefopam, la fluvoxamine, la clomipramine, la sertraline, la fluoxetine, le citalopram, l'escitalopram, la paroxetine, l'amitriptyline, la duloxetine, la venlafaxine, la buspirone, la carpipramine, le zolmitriptan, le sumatriptan, le naratriptan, l'indoramine, l'ergotamine, le tartrate d'ergotamine, le pizotifène, la pipamperone, le methysergide, la pizotyline, la tianeptine, le milnacipran, l'amitriptyline, la trimipramine, la viloxazine, la tianeptine, le millepertuis, le lithium.

Selon un autre mode de réalisation particulier, la drogue psychotrope est un effecteur opioïdergique tel que la nalbuphine, la buprenorphine, la pethidine, la codeine, le tramadol, la morphine, l'hydromorphone, l'oxycodone, la méthadone, le dextropropoxyphene, le meperidine, le fentanyl, le naltrexone, le chlorhydrate de morphine.

Selon un autre mode de réalisation particulier, la drogue psychotrope est un effecteur adénosinergique tel que la carbamazepine, l'oxcarbazepine.

Selon un autre mode de réalisation particulier, la drogue psychotrope est un effecteur ionotropique tel que la flunarizine, l'ethosuximide, le levetiracetam, la lamotrigine, la fosphenytoine, la phenytoine.

Selon un autre mode de réalisation particulier, la drogue psychotrope est un effecteur histaminergique tel que la niaprazine, l'hydroxyzine, la doxylamine.

Selon un autre mode de réalisation particulier, la drogue psychotrope est un effecteur Monoamine oxydase tel que le moclobemide, la selegiline, l'iproniazid.

Selon un autre mode de réalisation particulier, la drogue psychotrope est un effecteur catéchol-O-méthyl transférase tel que l'entacapone, le tolcapone.

Selon un autre mode de réalisation particulier, la drogue psychotrope est un effecteur DOPA decarboxylase tel que le benserazide, la carbidopa.

Selon un autre mode de réalisation particulier, la drogue psychotrope est un effecteur noradrenergique tel que la miansérine, la desipramine, le moclobémide ou le bupropion.

Selon un autre mode de réalisation particulier, la drogue psychotrope est un effecteur agissant au niveau du système limbique tel que la gabapentine, la captodiamine.

De manière encore plus préférée, la molécule psychotrope choisie est la clozapine, dérivé de dibenzodiazépine, ou bien la paroxetine, l'escitalopram, la sertraline, ou la venlafaxine, qui sont des effecteurs sérotinergique ou tout autre psychotrope effecteur sérotinergique tel que la chlorpromazine, la trimipramine, l'olanzapine, la cyamemazine, le flupentixol, le nefopam, la fluvoxamine, la clomipramine, la fluoxetine, le citalopram, l'amitriptyline, la duloxetine, la buspirone, la carpipramine, le zolmitriptan, le sumatriptan, le naratriptan, l'indoramine, le tartrate d'ergotamine, le pizotifène, la pipamperone, le methysergide, la pizotyline, la tianeptine.

De manière encore plus préférée, la molécule psychotrope choisie est le modafinil, dérivé de diphénylméthane, qui est un effecteur adrénergique ou tout autre psychotrope effecteur adrénergique tel que la dihydroergotamine, l'adrafinil, la mirtazapine, l'oxetorone.

Ainsi, de manière encore plus préférée, la molécule psychotrope est choisie parmi : le modafinil, la clozapine, la paroxetine, le diazepam, la venlafaxine, l'escitalopram, le bupropion, la sertraline.

Dans un mode de réalisation préféré, la molécule psychotrope est un antidépresseur choisi parmi : le moclobémide (MOCLAMINE), l'amitriptyline (LAROXYL), la clomipramine (ANAFRANIL), le milnacipran (IXEL), l'escitalopram (SEROPLEX), le citalopram (SEROPRAM), la fluoxétine (PROZAC), la paroxétine (DEROXAT), la fluvoxamine (FLOXYFRAL), la sertraline (ZOLOFT), la mirtapazine (NORSET), la duloxetine (Cymbalta) ou la venlafaxine (EFFEXOR) et le bupropion (ZYBAN).

Dans un mode de réalisation encore plus préféré, la molécule psychotrope est un antidépresseur choisi dans le groupe comprenant : la paroxétine, la venlafaxine, l'escitalopram, le bupropion et la sertraline.

Selon un troisième aspect, la présente invention concerne également l'utilisation de ce produit de combinaison, de façon simultanée, séparée ou étalée dans le temps, chez des patients souffrant de troubles psychiatriques et/ou neurodégénératifs.

Les patients ayant besoin de ce traitement peuvent présenter des troubles psychiatriques et/ou neurodégénératifs compris dans le groupe constitué par : la dépression, les maladies bi-polaires, l'épilepsie, la schizophrénie, l'anxiété généralisée, les états moroses, les maladies dues au stress, la panique, les phobies, les troubles obsessionnels compulsifs, les troubles comportementaux, la dépression du système immunitaire, la fatigue et les symptômes associés à la douleur, la fatigue chronique, la fibromyalgie, et d'autres troubles comme l'autisme, les baisses d'attention, l'hyperactivité, les maladies liées à l'alimentation comme la boulimie, l'anorexie, l'obésité, les troubles psychiques comme l'apathie, la migraine, la douleur, les maladies cardio-vasculaires, les maladies neurodégénératives et associées à l'anxiété dépressive (maladie d'Alzheimer, Chorée de Huntigton, maladie de Parkinson), toxicomanie et addiction aux drogues.

Dans le cas d'une utilisation simultanée, les deux composantes du traitement combiné sont administrées au patient simultanément. Selon ce mode de mise en oeuvre de la présente invention, les deux composantes peuvent être conditionnées ensemble, sous forme de mélange, ou bien conditionnées séparément, puis mélangées extemporanément avant d'être administrées ensemble au patient. Plus communément, les deux composantes sont administrées simultanément mais de manière séparée. En particulier, les voies d'administration des deux composantes peuvent être différentes. L'administration peut aussi se faire à des sites différents. Dans un autre mode de mise en oeuvre, les deux composantes sont administrées de manière étalée ou espacée dans le temps, par exemple dans la même journée ou à un intervalle allant de quelques jours à quelques semaines, voire quelques mois.

Selon un quatrième aspect, la présente invention prévoit l'utilisation d'au moins un agent bloquant les connexines pour préparer un médicament destiné à être administré avant, en même temps ou après une drogue psychotrope, pour traiter un patient souffrant de troubles psychiatriques et/ou neurodégénératifs.

Selon un cinquième aspect, l'invention comprend l'utilisation d'au moins un agent bloquant les connexines, pour moduler et/ou potentialiser l'effet d'une drogue psychotrope chez des patients souffrant de troubles psychiatriques et/ou neurodégénératifs.

Le terme « moduler » signifie ici intervenir par la potentialisation ou l'antagonisme des effets directes ou indirectes de la drogue psychotrope administrée avant, simultanément ou après l'agent anti-connexine, notamment au niveau des effets secondaires.

Le terme « potentialiser » signifie ici augmenter significativement les effets de la drogue psychotrope administrée avant, simultanément ou après l'agent anti-connexine. Ainsi, l'association de la drogue psychotrope avec l'agent anti-connexine permet de diminuer les doses de ladite drogue psychotrope et donc de limiter les effets secondaires de ladite drogue psychotrope, et/ou de réduire les effets d'échappement et de sevrage.

L'invention concerne donc également l'utilisation d'au moins un agent bloquant les connexines, pour diminuer les doses de ladite drogue psychotrope et/ou limiter les effets secondaires de ladite drogue psychotrope, et/ou réduire les effets d'échappement et de sevrage.

Selon un dernier aspect, l'invention décrit une méthode pour traiter un patient souffrant de troubles psychiatriques et/ou neurodégénératifs, comprenant l'administration à ce patient de :

a) au moins une substance active sélectionnée parmi les drogues psychotropes, et
b) au moins un agent bloquant les connexines,

et dans laquelle lesdits produits a) et b) sont administrés simultanément, séparément, ou de façon séparée dans le temps.

**Exemples**

L'analyse des domaines du cerveau par électroencéphalographie est aujourd'hui considérée comme une technique fiable et très sensible pour caractériser les effets d'une drogue sur le système nerveux central. L'électro-encéphalographie (EEG) est la mesure de l'activité électrique du cerveau par des électrodes placées sur le cuir chevelu, souvent représenté sous la forme d'un tracé appelé électro-encéphalogramme. Comparable à l'électrocardiogramme qui permet d'étudier le fonctionnement du coeur, l'EEG est un examen indolore et non-invasif qui renseigne sur l'activité neurophysiologique du cerveau au cours du temps et en particulier .du cortex cérébral soit dans un but diagnostique en neurologie, soit dans la recherche en neurosciences cognitives. Le signal électrique à la base de l'EEG est la résultante, pour chaque fréquence, de la sommation des potentiels d'action post-synaptiques synchrones issus d'un grand nombre de neurones.

Il a été démontré que la puissance électrique associée à chaque fréquence peut varier indépendamment de celle des autres en fonction du comportement de l'individu ou la drogue administrée (Dimpfel W, Neuropsychobiology. 1986;15(2):101-8). Après l'administration d'une drogue, l'électroencéphalogramme du patient change, et la répartition des potentiels associés à chaque fréquence constitue l'électropharmacogramme de cette drogue. En général, les électropharmacogrammes sont de natures différentes pour des drogues prescrites pour des maladies différentes, et sont similaires lorsqu'elles visent à traiter des pathologies identiques (Dimpfel W, British Journal of Pharmacology 2007, 152, 538-548). Les électropharmacogrammes correspondant à plus de 150 drogues ont été déterminés (par exemple, analgésiques, antidépresseurs, neuroleptiques, stimulants, tranquillisants, sédatifs et narcotiques). Aujourd'hui, suite à ces nombreuses études, la détermination et l'analyse de l'électropharmacogramme est considérée comme une technique fiable pour mesurer les effets pharmacologiques d'une drogue. De plus, les paramètres de l'EEG permettent d'obtenir des informations quantitatives sur le développement en phase clinique de nombreux composés (Mandema & Danhof, Clin. Pharmacokinet. 1992, 23, 191-215). La mesure du potentiel EEG peut être également utilisée pour identifier les récepteurs cellulaires des drogues administrées (Parker TJ, British Journal of Pharmacology 2001, 132, 151-158). L'EEG est une mesure de l'activité électrique du cerveau et il existe différents modes de représentations des données électroencéphalographiques. La première est la représentation des tracés et l'identification de phénomènes ondulatoires caractéristiques. Cette donnée qualitative est informative pour des épisodes d'activité électrique bien déterminés, mais ne renseigne pas sur l'aspect quantitatif de l'activité électrique. Pour cela, l'expérimentateur fait appel à l'EEG quantitatif basé sur l'analyse du signal par transformée de Fourier permettant d'obtenir des valeurs de puissance pour une fréquence donnée au cours du temps. Cette valeur de puissance est rapportée à une valeur contrôle qui permet de déterminer la modification de puissance pour une fréquence donnée au cours du temps. Cette valeur peut-être moyennée par des périodes de temps variables selon les expériences. Ces variations de puissance au cours du temps peuvent-être représentées selon les auteurs soit sous forme de moyennes sur des plages spécifiques de fréquences correspondant à des rythmes physiologiques ou pathologiques (Delta (1-4Hz), Theta (4,5-8Hz), Alpha (8,5-12Hz) Beta (12,5-24Hz) et Gamma (>24Hz)) , soit sous forme d'histogrammes de puissances relatives Hz par Hz. Ces deux modes de représentations sont strictement équivalents, l'un découlant de l'autre. (voir EEG : Bases neurophysiologiques, principes d'interprétation et de prescription Jean Vion-Dury, France Blanquet. Editeur: MASSON ; Collection : Abrégé Masson). Dans les exemples présentés plus loin, la représentation des puissances relatives Hz par Hz a été choisie.

En ce qui concerne par exemple les antipsychotiques atypiques, et en particulier la clozapine, il a été montré que leur administration induit un électropharmacogramme bi-phasique dans le temps : au cours de la première heure, le potentiel EEG est très élevé pour les fréquences très faibles (entre 0,8 et 4,5 Hz) (rythme delta) et entre 7 et 9,5 Hz (rythme alpha 1), et d'intensité faible dans les fréquences entre 4,75 et 6,75 Hz (rythme theta) et supérieures à 18,5 Hz (rythme beta), ce qui est le signe d'une réponse clinique positive (Galderisi S, Methods Find Exp Clin Pharmacol, 2002, 24, 85-89). Au cours de la deuxième heure, le potentiel EEG est d'intensité moyenne sur les fréquences allant de 8 à 15 Hz, ce qui peut impliquer des effets secondaires extrapyramidaux. En effet, un potentiel EEG d'intensité moyenne dans la deuxième heure pour les fréquences 7-9,5 Hz et 12,75-18,50 Hz, indique que les effets secondaires seront présents (Dimpfel W, British Journal of Pharmacology 2007, 152, 538-548).

**Protocole :**

*- Pré-implantation des électrodes*

Un lot de 6 rats Wistar vigils a été pré-implanté avec 6 électrodes bipolaires bilatérales (2 frontales, 2 hippocampiques antérieures, 2 hippocampiques postérieures).

*- Injections*

Différents traitements ont été réalisés par combinaison circulaire de 6 rats par traitement (traitement psychotrope seul, traitement anticonnexine seul, traitement association anticonnexine+psychotrope). L'injection de l'acide Méclofénamique (Sigma) se fait par injection lente sous méningée (80ng/kg, 4,5pg/seconde) ou en intra péritonéal à différentes doses. L'injection de l'acide glycyrrhétinique (Sigma) se fait en injection sous méningée (80ng/kg). L'injection de Clozapine (Sigma) se fait par voie intra-péritonéale (0,2 mg/kg). L'injection de Paroxetine (Sigma) se fait par voie intra-péritonéale (0,5mg/kg). L'injection de Modafinil (Cephalon) se fait par voie intra-péritonéale (125 mg/kg et 250 mg/kg)

*- Mesure de l'EEG*

Les mesures de l'EEG ont été réalisées sur les différents lots de rats vigiles (préalablement implantés et habitués) par des enregistrements de 2 heures après injection. L'analyse spectrale réalisée par transformée de Fourier (FFT) permet l'obtention des puissances relatives Hertz par Hertz et seconde par seconde. Les données de FFT sont ensuite moyennées minute par minute et rapportée au contrôle solvant effectué la veille de l'enregistrement dans des conditions expérimentales strictement identiques. Les puissances spectrales relatives des cortex préfrontaux gauche et droite sont ensuite moyennées par période de 5 minutes, moyennées par groupe de 12 répétitions de 5 minutes et représentées heure par heure.

*- Analyses Statistiques :*

Les données issues de l'analyse quantitative relative d'EEG des effets des différentes drogues administrées individuellement (psychotropes seuls ou anticonnexines seuls), présentées en exemples, ont été soumises à une analyse statistique par analyse de Variance (ANOVA) à trois facteurs : « Fréquence » (par Hz), « Temps » (première et deuxième heure par 12 répétitions de 5 minutes moyennées), et « Animal » (6 animaux différents pour chaque traitement). Pour les associations de psychotropes et anticonnexines présentées en exemples, un quatrième facteur d'ANOVA a été introduit : « Association anticonnexine ». Le seuil de significativité des modifications de puissance relative pour une fréquence donnée pour une drogue psychotrope seule par rapport au contrôle (solvant du psychotrope) ou une drogue psychotrope en association avec un anticonnexine par rapport au psychotrope administré seul (en faveur d'une augmentation ou d'une diminution de puissance relative) a été choisi à une valeur de $P<0,05$.

**Exemple 1 : Effets d'inhibiteurs de connexines**

Une série d'expériences pour étudier l'influence des connexines dans l'activité EEG de différentes structures du cerveau a été réalisée. Pour cela les connexines cérébrales ont été inhibées, par administration lente, directement sous-méningée, d'un inhibiteur des connexines, l'Acide méclofénamique (MFA). Dans les mêmes conditions expérimentales, les effets de l'administration intra-cérébrale de l'acide glycyrrhétinique (BGA), un autre agent anticonnexine, ont été étudiés.

L'analyse de l'activité électrique cérébrale chez les rats traités avec l'inhibiteur de connexine (MFA) par voie intracérébrale met en évidence une augmentation significative du potentiel EEG et donc de la synchronisation de l'activité cérébrale préfrontale (140%) pour les fréquences 8-10Hz, à la première heure et s'étendant à la deuxième heure. Des modifications significatives similaires sont observées lors de l'injection d'une autre classe d'anticonnexine (acide Glycyrrhétinique) par voie intracérébrale (figure 1).

Cette synchronisation indique que les connexines ont un rôle physiologique de désynchronisation des activités électriques. Compte tenu de l'effet global des inhibiteurs de connexines sur les différentes régions étudiées et la présence ubiquitaire des jonctions composées de connexines dans le cerveau, ces résultats expérimentaux permettent de réinterpréter les données de la littérature et de proposer un rôle inédit des connexines dans la normalisation des activités électrophysiologiques du cerveau. Cette normalisation aurait pour but de circonscrire les phénomènes d'emballement de l'activité électrique et de moduler et d'amortir les dysfonctionnements anarchiques du système hautement interconnecté.

**Exemple 2: Effet d'un antipsychotique atypique en combinaison avec un inhibiteur de connexines**

Afin d'explorer l'hypothèse d'un rôle modulateur global des connexines dans l'activité cérébrale, les conséquences électrophysiologiques d'un traitement pharmacologique associé à des molécules anti-connexines ont été étudiées.

Dans ce cadre, les effets de la Clozapine en association avec un inhibiteur des connexines, l'acide méclofénamique (MFA), ont été étudiés. La Clozapine est un antipsychotique atypique indiqué dans les schizophrénies, antagoniste des récepteurs sérotoninergiques et dopaminergiques, accessoirement antagoniste adrénergique, cholinergique et histaminergique et présentant un spectre typique d'activation EEG complexe (Parker TJ, British Journal of Pharmacology 2001, 132, 151-158), et une liste considérable d'effets indésirables aux doses thérapeutiques (prise de poids, diminution de moelle osseuse et du nombre de globules blancs dans le sang).

### 2.1 Effet de la Clozapine

Dans un premier temps, le modèle expérimental a été validé pour vérifier qu'il était en accord avec les données publiées (Dimpfel W, British Journal of Pharmacology 2007, 152, 538-548, Parker TJ, British Journal of Pharmacology 2001, 132, 151-158). Des enregistrements de l'effet de la clozapine seule ont donc été réalisés.

L'analyse spectrale des EEG met en évidence une augmentation significative du potentiel EEG, et donc de la synchronisation de l'activité cérébrale préfrontale (200%) pour les fréquences 8-10 Hz et dans une plus faible mesure pour les fréquences 16-20 Hz lors du traitement avec l'antipsychotique seul, dès la première heure et s'étendant à la deuxième heure (figure 2).

Ces observations sont parfaitement en accord avec les effets caractéristiques d'antipsychotique atypique rapportés dans la littérature (Dimpfel W, British Journal of Pharmacology 2007, 152, 538-548, Parker TJ, British Journal of Pharmacology 2001, 132, 151-158).

### 2.2 Effet de l'association Clozapine et Anticonnexine

Dans un second temps, l'influence du système jonctionnel des connexines sur l'effet électrophysiologique de la clozapine été étudiée.

Lors du traitement combinant l'antipsychotique (clozapine) et l'inhibiteur de connexine (MFA), l'analyse spectrale des EEG met en évidence une augmentation significative de la synchronisation de l'activité cérébrale préfrontale (260%) pour les fréquences 8-10 Hz et dans une plus faible mesure pour les fréquences 16-20 Hz dès la première heure. Cette augmentation significative de la synchronisation correspond au spectre de la clozapine et correspond donc à une potentialisation de l'effet de la clozapine par le blocage du système jonctionnel des connexines. Ce mécanisme de renforcement de l'effet de la clozapine par l'anticonnexine est contrôlé dans le temps. En effet, à la deuxième heure ne persiste que l'effet d'anticonnexine (figure 2).

En outre, en focalisant l'analyse sur une fréquence significative (la fréquence 8 Hz par exemple), le renforcement de l'effet de la clozapine observé s'accompagne d'un amortissement considérable de l'amplitude des effets d'oscillation (figure 3), alors que le traitement avec la clozapine seule entraîne des fluctuations d'amplitude importante (figure 3).

Ainsi, l'amortissement des fluctuations induit par le blocage du système jonctionnel des connexines indique que les connexines pourraient jouer un rôle dans la mise en place de ces fluctuations brèves de l'activité électrophysiologique. Ces fluctuations correspondraient à des mécanismes rapides d'embrasement de l'activité par la clozapine et contrôlées par les connexines.

En définitive, ces résultats confirment l'hypothèse d'un rôle modulateur (en intensité globale et sur des fluctuations locales et temporelles) des activités électrophysiologiques par le système jonctionnel des connexines. Par ailleurs, ces observations démontrent une modulation inédite de l'effet de l'antipsychotique par sa combinaison avec un inhibiteur de connexine. Cette modulation se manifeste par la potentialisation significative et la stabilisation de l'effet de l'antipsychotique sur une période plus courte. Cette potentialisation de l'effet de l'antipsychotique serait identique à celle obtenue avec une dose plus importante de clozapine mais avec un contrôle sur la durée d'action.

### Exemple 3 : Comparaisons des voies d'administration

Les conséquences électrophysiologiques du blocage des connexines selon la voie d'administration centrale ou périphérique (intracérébrale vs intra-péritonéale) de l'inhibiteur MFA ont été comparées.

L'analyse spectrale des EEG, évaluant les voies d'administration périphérique et centrale, met en évidence une augmentation significative comparable de la synchronisation de l'activité cérébrale préfrontale dès la première heure et ce pour des plages de fréquences similaires. En revanche, l'augmentation significative de synchronisation se poursuit à la deuxième heure pour l'administration périphérique alors qu'elle diminue pour l'administration centrale. Cette différence à la deuxième heure est liée à la pharmacocinétique des voies d'administration (figure 4).

En définitive, ces résultats préliminaires indiquent que la voie d'administration, qu'elle soit intracérébrale ou périphérique, ne modifie l'effet que dans son amplitude.

### Exemple 4 : Effet de différentes doses d'anticonnexine par voie intrapéritonéale

Les conséquences électrophysiologiques du blocage des connexines selon la dose administrée par voie périphérique de l'inhibiteur MFA ont été comparées (effet dose).

L'analyse spectrale des EEG, évaluant les doses administrées par voie périphérique, met en évidence des variations significatives importantes de la synchronisation sur différentes plages de fréquences selon la dose. Ces modifications en faveur d'une synchronisation de l'activité cérébrale préfrontale apparaissent dès la première heure et se poursuivent à la deuxième heure de façon variable selon la dose. Cette synchronisation par inhibition des connexines confirme le caractère désynchronisateur physiologique des connexines (Figure 5). Ces variations importantes de synchronisation suivant la dose peuvent être dues à la représentation quantitative (nombre de jonctions lacunaires variable selon le réseau du système nerveux centrale, ex : Cholinergique, Noradrénergique, Serotoninergique etc.) et/ou qualitative (affinité des différentes isoformes de connexines pour l'inhibiteur à la dose donnée) des connexines.

En définitive, ces résultats préliminaires indiquent que les effets propres des anticonnexines administrées par voie intrapéritonéale dépendent de la dose. Ceci semble indiquer que ce système de modulation via les connexines, même si il est ubiquitaire dans le cerveau, présente des spécificités locales (d'isoforme ou de quantité) dans le cerveau et ce en accord avec la littérature (Fukuda T., Neuroscientist 2007 ; 13(3)199-207). Ceci indique en outre que selon le système ciblé spécifiquement par la drogue psychotrope (cholinergique, serotoninergique, noradrénergique, gabaergique etc.), selon sa structure et ses propriétés chimiques, selon sa pharmacologie (demi-vie, métabolisme, clearance), la dose d'anticonnexine devra sans doute être adaptée.

Enfin, cette étude effet-dose a permis d'identifier la dose de MFA administrée par voie intrapéritonéale qui entraîne le minimum d'effet EEG propre (pas de modification significative à la première heure, légère augmentation significative à la deuxième heure), compatible avec l'exploration de potentialisation des effets de différents psychotropes. Cette dose de MFA est de 0,4mg/kg (dose 10 à 25 fois inférieure à la dose utilisée pour des effets anti-inflammatoires) et sera la dose utilisée pour les exemples de potentialisation décrits en exemple 5 et 6.

### Exemple 5 : Effet d'un premier antidépresseur en combinaison avec un inhibiteur de connexines

Afin de poursuivre l'évaluation de l'hypothèse d'un rôle modulateur global des connexines dans l'activité cérébrale, les conséquences électrophysiologiques d'un premier traitement pharmacologique antidépresseur associé à des molécules anti-connexines ont été étudiées.

Dans ce cadre, les effets de la Paroxetine en association avec un inhibiteur des connexines, l'acide méclofénamique (MFA), ont été étudiés. La Paroxetine est un antidépresseur, indiqué dans les épisodes dépressifs aigues ou chroniques, dérivé de la phénylpipéridine du groupe des inhibiteurs sélectifs de la recapture de la sérotonine et présentant une liste considérable d'effets indésirables aux doses thérapeutiques (apathie, dilatation de la pupille, nausées, tératogenicité, somnolence, maux de tête, modifications du poids et de l'appétit, changements du comportement sexuel, augmentation des sentiments de dépression et d'anxiété , sécheresse de la bouche, comportement agressif (surtout chez les enfants), malformations congénitales possibles, érythème, instabilité psychomotrice, démangeaisons, déplétion (Sodium), sueurs, idées suicidaires, faiblesse musculaire, douleurs musculaires, niveaux d'agression inhabituels, syndrome sérotoninergique).

### 5.1. Effet de la Paroxetine

Dans un premier temps, le modèle expérimental a été évalué dans la mesure où aucune donnée bibliographique sur l'effet EEG de la paroxetine chez le rat vigile n'est disponible. En revanche, différentes études sur les effets de la paroxetine notamment sur les rythmes veille sommeil fournissent les doses pharmacologiques de paroxetine en traitement aigue qui sont généralement de 2 à 5 mg/kg (Sanchez C., Pharmacol Biochem Behav. 2007 Mar;86(3):468-76). Des enregistrements de l'effet de la paroxetine seule ont donc été réalisés à la dose de 0,5 mg/kg administrée par voie intrapéritonéale (4 à 10 fois inférieures à la dose habituellement utilisée en traitement aigu chez le rat).

L'analyse spectrale des EEG met en évidence une augmentation significative des puissances relatives dans le cortex préfrontal, et donc de la synchronisation de l'activité cérébrale préfrontale (environ 200%) 8-10 Hz et dans une plus faible mesure pour les pour les fréquences 2-3 Hz et 18-19 Hz lors du traitement avec l'antidépresseur seul, dès la première heure et augmentant (environ 300%) à la deuxième heure pour les fréquences 8-10 Hz et dans une plus faible mesure pour les fréquences 2-3 Hz et 18-19 Hz (figure 6).

### 5.2. Effet de l'association Paroxetine et Anticonnexine

Dans un second temps, l'influence du système jonctionnel des connexines sur l'effet électrophysio logique de la Paroxetine a été étudiée.

Lors du traitement combinant l'antidépresseur (Paroxetine) et l'inhibiteur de connexine (MFA), l'analyse spectrale des EEG met en évidence une augmentation significative de la synchronisation de l'activité cérébrale préfrontale (600%) pour les fréquences 8-10 Hz et dans une plus faible mesure pour les fréquences 2-3 Hz et 18-19 Hz à la deuxième heure. Cette augmentation significative de la synchronisation correspond parfaitement au spectre de la paroxetine seule et correspond donc à une potentialisation considérable de l'effet de la paroxetine par le blocage du système jonctionnel des connexines. En outre, ce mécanisme de renforcement de l'effet de la paroxetine par l'anticonnexine suit la même évolution temporelle que la paroxetine seule. En effet, la paroxetine seule ou en association avec l'anticonnexine entraîne des effets EEG qui augmentent à la deuxième heure et qui doivent s'estomper plusieurs heures après administration (figure 6).

Ainsi, à la différence de la clozapine, qui elle entraîne un maximum d'activité EEG dès la première heure, la paroxetine entraîne des effets plus prolongés dans le temps et qui mettent plus de temps à s'installer. La potentialisation par l'anticonnexine conserve donc les propriétés d'évolution temporelles des molécules psychotropes étudiées (de natures chimiques différentes, ciblant des systèmes différents et d'indication très différentes) et renforce la notion d'un système de modulation par les connexines non limité à un seul système de neurotransmission.

En définitive, ces résultats confirment l'hypothèse d'un rôle modulateur (en intensité globale et sur l'évolution temporelle) des activités électrophysiologiques par le système jonctionnel des connexines.

Enfin, ces observations démontrent une modulation inédite de l'effet de l'antidépresseur par sa combinaison avec un inhibiteur de connexine. Cette modulation se manifeste par la potentialisation de l'effet de l'antidépresseur à une dose 4 à 25 fois moins importantes que la dose pharmacologique. Cette potentialisation de l'effet de l'antidépresseur par l'anticonnexine permettrait donc de réduire les doses d'antidépresseur de 4 à 25 fois et ce avec le minimum d'effet propre de l'anticonnexine. Le bénéfice de dose mesuré de l'association paroxetine et anticonnexine serait donc supérieur à 4.

### Exemple 6 : Effet d'un psychostimulant en combinaison avec un inhibiteur de connexines

Afin de poursuivre l'évaluation de l'hypothèse d'un rôle modulateur global des connexines dans l'activité cérébrale, les conséquences électrophysiologiques d'un traitement pharmacologique psychostimulant associé à des molécules anti-connexines ont été étudiées.

Dans ce cadre, les effets du Modafinil (Provigil) en association avec un inhibiteur des connexines, l'acide méclofénamique (MFA), ont été étudiés. Le Modafinil est un psychostimulant, indiqué dans le traitement de la narcolepsie et de l'hypersomnie idiopathique, inhibiteur de la recapture de la Noradrénaline et présentant une liste d'effets indésirables aux doses thérapeutiques (excitation, agressivité, insomnie, anorexie, maux de tête, nausées, gastralgies, éruptions cutanées allergiques).

### 6.1 Effet du Modafinil

Dans un premier temps, le modèle expérimental a été évalué avec les données publiées qui fournissent notamment les doses pharmacologiques de Modafinil en traitement aigue qui sont généralement de 100 à 350 mg/kg (Sebban C., British Journal of Pharmacology (1999) 128, 1045-1054, De saint Hilaire Z., Neuroreport. 2001 Nov 16;12 (16):3533-7). Des enregistrements de l'effet du modafinil seul à deux doses (125 et 250 mg/kg) ont donc été réalisés.

L'analyse spectrale des EEG met en évidence une augmentation significative du potentiel EEG, et donc de la synchronisation de l'activité cérébrale préfrontale (300% pour la dose 250 mg/kg et 120% pour la dose 125mg/kg) pour les fréquences 2-5 Hz et une diminution significative du potentiel EEG, et donc une désynchronisation de l'activité cérébrale préfrontale (10 à 30%) pour les fréquences 10-25 Hz pour la dose 125 mg/kg et dans une plus faible mesure pour la dose de 250 mg/kg pour le traitement avec le psychostimulant seul, dès la première heure et augmentant à la deuxième heure (figure 7).

Ces observations sont compatible avec les effets caractéristiques de psychostimulant rapportés dans la littérature (Sebban C., British Journal of Pharmacology (1999) 128, 1045-1054, De Saint Hilaire Z., Neuroreport. 2001 Nov 16;12(16):3533-7).

### 6.2. Effet de l'association Modafinil et Anticonnexine

Dans un second temps, l'influence du système jonctionnel des connexines sur les effets électrophysiologiques du Modafinil à dose faible (125 mg/kg) a été étudiée.

Lors du traitement combinant le psychostimulant (Modafinil) et l'inhibiteur de connexine (MFA), l'analyse spectrale des EEG met en évidence une augmentation significative de la synchronisation de l'activité cérébrale préfrontale (300%) pour les fréquences 2-5 Hz et dans une plus faible mesure une diminution significative du potentiel EEG, et donc une désynchronisation de l'activité cérébrale préfrontale pour les fréquences 10-25 Hz dès la première heure et augmentant à la deuxième heure (figure 7). Ces modifications EEG correspondent parfaitement au spectre du Modafinil seul à la dose forte (250 mg/kg) et correspondent donc à une potentialisation de l'effet du Modafinil par le blocage du système jonctionnel des connexines. En outre, à l'instar de la Clozapine et de la Paroxetine, ce mécanisme de renforcement de l'effet du Modafinil par l'anticonnexine suit la même évolution temporelle que le modafinil seul à une dose supérieure. Cette potentialisation par l'anticonnexine conservant les propriétés d'évolution temporelles des psychotropes de nature très différente confirme à nouveau la notion d'un système de modulation par les connexines non limité à un seul système de neurotransmission.

En définitive, ces résultats confirment l'hypothèse d'un rôle modulateur (en intensité globale et sur l'évolution temporelle) des activités électrophysiologiques par le système jonctionnel des connexines. Par ailleurs, ces observations démontrent une modulation inédite de l'effet de psychostimulant par la combinaison avec un inhibiteur de connexine. Cette modulation se manifeste par la potentialisation de l'effet du psychostimulant à une dose faible et mime parfaitement les effets d'une dose forte. Cette potentialisation de l'effet du psychostimulant par l'anticonnexine permettrait donc de réduire les doses de psychostimulant d'un facteur au moins deux et ce sans effet propre de l'anticonnexine. Le bénéfice de dose mesuré de l'association modafinil et anticonnexine serait donc d'au moins deux.

## Exemple 7 : Effet d'un anxiolytique en combinaison avec un inhibiteur de connexines

Afin de poursuivre l'évaluation de l'hypothèse d'un rôle modulateur global des connexines dans l'activité cérébrale, les conséquences électrophysiologiques d'un traitement pharmacologique anxiolytique associé à une molécule anti-connexine ont été étudiées.

Dans ce cadre, les effets du Diazépam en association avec un inhibiteur des connexines, l'acide méclofénamique (MFA) ont été étudiés. Le Diazépam est un anxiolytique, indiqué dans les épisodes d'anxiété excessive, les insomnies d'endormissement, les états névrotiques, manifestations psychosomatiques, les désintoxications alcooliques et l'épilepsie. Le Diazépam est une benzodiazépine facilitateur de la transmission Gabaergique, présentant une liste considérable d'effets indésirables aux doses thérapeutiques (somnolence, hypotonie, sensations ébrieuses, difficultés de concentration, irritabilité, agressivité, excitation, confusion, hépatite, réactions cutanées allergiques, dysphagie) et peut entraîner une tolérance et dépendance nécessitant l'adaptation de la posologie pendant les différentes phases du traitement.

### 7.1. Effet du Diazépam

Dans un premier temps le modèle expérimental a été évalué pour le comparer aux données bibliographique sur l'effet EEG du Diazépam par voie intra péritonéale chez le rat vigile (Robledo P., Alcohol Clin Exp Res. 1994 Apr; 18(2)-363-8). Des enregistrements de l'effet du Diazépam seul ont donc été réalisés à la dose de 1mg/kg administrée par voie intrapéritonéale (1,5 à 5 fois inférieures à la dose habituellement utilisée en traitement aigu chez le rat).

L'analyse spectrale des EEG met en évidence, dès la première heure, les effets caractéristiques du Diazépam à savoir une légère diminution de puissance des composantes de 4 à 7 Hz (environ 25% de diminution) et une augmentation significative de puissance des composantes de 11 à 30Hz (environ 120%). A la deuxième heure, ces effets sur le 4-7Hz et le 11-30Hz perdurent en diminuant en intensité et une augmentation significative de puissance des composantes 1-3Hz apparaît (cf. figure 8).

### 7.2. Effet de l'association Diazépam et Anticonnexine

Après avoir validé le modèle expérimental, l'influence du système jonctionnel des connexines sur l'effet électrophysiologique du Diazépam a été étudiée.

Lors du traitement combinant l'anxiolytique (Diazépam, 1mg/kg par voie intrapéritonéale) et l'inhibiteur de connexine (MFA, 0,4 mg/kg par voie intrapéritonéale), l'analyse spectrale des EEG met en évidence, dès la première heure, une augmentation significative de puissance pour les composantes de 11 à 30Hz (environ 150%) et les composantes 1-3Hz (environ 120%). Cette augmentation de puissance se poursuit à la deuxième heure avec une plus forte amplitude pour les composantes de 11 à 30Hz (jusqu'à 200%) (cf.figure 8).

Cette augmentation significative de puissance des composantes 1-3Hz et 11-30Hz correspond au spectre du diazépam seul à des doses plus fortes (de 2 à 10 fois la dose utilisée dans le premier test) et correspond donc à une potentialisation considérable de l'effet du diazépam par le blocage du système jonctionnel des connexines. En outre, à la différence du diazépam seul, la potentialisation par l'anticonnexine renforce l'effet à la deuxième heure indiquant une action prolongée dans le temps en présence d'anticonnexine. Cette dernière observation se distingue de l'effet des molécules anticonnexines en combinaison avec la Clozapine, le modafinil et la paroxetine en ne suivant pas exactement l'évolution temporelle du Diazépam seule, indiquant que le système de modulation par les connexines peut être quantitativement différent d'un système de neurotransmission à l'autre. Ainsi, dans le cas du Diazépam, le blocage du système jonctionnel aurait une influence plus forte à la deuxième heure qu'à la première, soulignant que selon l'organisation du système jonctionnel au sein des systèmes de neurotransmission chimique, son blocage aura des conséquences qualitatives et quantitatives différentes.

En définitive, cette potentialisation du système Gabaergique confirme l'hypothèse d'un rôle modulateur (vraisemblablement qualitativement et quantitativement dépendant de son organisation au sein des systèmes de neurotransmission chimique) des activités électrophysiologiques par le système jonctionel des connexines.

Enfin, ces résultats démontrent une modulation inédite de l'effet d'anxiolytique par la combinaison avec un inhibiteur de connexine. Cette modulation se manifeste par une potentialisation de l'effet de l'anxiolytique qui, à une dose 2 à 10 fois moins importante que la dose pharmacologique, permet d'obtenir, lorsqu'il est associé à la molécule anticonnexine, le même effet électroencéphalographique que la dose pharmacologique seule. Cette potentialisation de l'effet de l'anxiolytique par l'anticonnexine permettrait donc de réduire les doses d'anxiolytique de 2 à 10 fois et ce avec le minimum d'effet propre de l'anticonnexine. Le bénéfice de dose mesuré de l'association Diazépam et l'anticonnexine serait donc supérieur à 2.

**Exemple 8 : Effet d'un second antidépresseur en combinaison avec un inhibiteur de connexines**

Afin de poursuivre l'évaluation de l'hypothèse d'un rôle modulateur global des connexines dans l'activité cérébrale, les conséquences électrophysiologiques d'un second traitement pharmacologique antidépresseur associé à des molécules anti-connexines ont été étudiées.

Dans ce cadre, les effets de la Venlafaxine en association avec un inhibiteur des connexines, l'acide méclofénamique (MFA) ont été étudiés. La Venlafaxine est un antidépresseur, indiqué dans les épisodes dépressifs majeurs de l'adulte, la prévention de récidives dépressives chez les patients présentant un trouble unipolaire, et l'anxiété généralisée depuis au moins 6 mois chez l'adulte. La Venlafaxine est un inhibiteur de la recapture de la sérotonine et de la noradrénaline, de profil intermédiaire, d'efficacité comparable à celle des imipraminiques, présentant une liste considérable d'effets indésirables aux doses thérapeutiques (nausées, somnolence, bouche sèche, insomnie, vertige, constipation, sueurs, hyponatrémie, trouble de l'éjaculation, diarrhée, vomissement, prise de poids, céphalées, agitation, tremblements, paresthésies, palpitation, trouble de l'accommodation, éruptions cutanées) et peut entraîner un syndrome de sevrage à l'arrêt du traitement.

**8.1 Effet de la Venlafaxine**

Dans un premier temps le modèle expérimental a été évalué avec la Venlafaxine seule, dans la mesure où aucune donnée bibliographique sur l'effet EEG de la Venlafaxine chez le rat vigile n'est disponible ; en revanche, différentes études sur les effets de la venlafaxine notamment sur les modifications du sommeil fournissent les doses pharmacologiques de venlafaxine en traitement aigu, qui sont généralement de 1 à 10mg/kg par voie intrapéritonéale (Salin-Pascual RJ., Psychopharmacology. 1997 Feb ; 129(3) :295-6).

Des enregistrements de l'effet de la venlafaxine seule ont été réalisés à deux doses, 0,16mg/kg et 4mg/kg administrée par voie intrapéritonéale (1 à 25 fois inférieures à la dose habituellement utilisée en traitement aigu chez le rat).

L'analyse spectrale des EEG préfrontaux met en évidence une augmentation significative de puissance, selon la dose de venlafaxine, des composantes de 8 à 10Hz (environ 120% pour la dose 0,16mg/kg et 200% pour la dose 4mg/kg) et des composantes de 16-18Hz et dans une moindre mesure les composantes de 24-26Hz dès la première heure et augmentant à la deuxième heure avec apparition d'une augmentation de puissance des composantes 1-3Hz à 4mg/kg (figure 9).

**8.2. Effet de l'association Diazépam et Anticonnexine**

Par la suite, l'influence du système jonctionnel des connexines sur l'effet électrophysiologique de la Venlafaxine a été étudiée.

Lors du traitement combinant l'antidépresseur (Venlafaxine, 0,16 mg/kg par voie intrapéritonéale) et l'inhibiteur de connexine (MFA, 0,4mg/kg par voie intrapéritonéale), l'analyse spectrale des EEG met en évidence, dès la première heure, une augmentation significative de puissance pour les composantes de 8 à 10Hz (environ 350%), les composantes 16-18Hz (environ 200%) et les composantes 24-26Hz (environ 180%). Cette augmentation de puissance se poursuit à la deuxième heure avec diminution d'amplitude dans les différentes composantes (majoritairement pour les composantes 8-10Hz) sauf pour les composantes 1-3Hz pour lesquelles une augmentation significative de puissance est observée (cf. figure 9).

Cette augmentation significative de la synchronisation correspond au spectre de la venlafaxine seule et correspond donc à une potentialisation considérable de l'effet de la venlafaxine par le blocage du système jonctionnel des connexines. Par ailleurs, à la différence de la venlafaxine seule, la potentialisation par l'anticonnexine renforce l'effet à la première heure et diminue à la deuxième (tout en restant supérieur au traitement venlafaxine seul) indiquant une action rapide qui se prolonge dans le temps en présence d'anticonnexine. Cette dernière observation se rapproche de l'observation des résultats de l'association avec le Diazepam et se distingue de l'effet des anticonnexines en combinaison avec la Clozapine, le modafinil et la paroxetine en ne suivant pas exactement l'évolution temporelle de la venlafaxine seule, confirmant que le système de modulation par les connexines peut être quantitativement différent d'un système de neurotransmission à l'autre. Ainsi, dans le cas de la venlafaxine, se confirme la notion que le blocage du système jonctionnel aura des conséquences qualitatives et quantitatives différentes selon son organisation au sein des systèmes de neurotransmission chimique.

En définitive, cette potentialisation des systèmes Serotoninergique et Noradrénergique confirme l'hypothèse d'un rôle modulateur majeur des activités électrophysiologiques par le système jonctionnel des connexines.

Enfin, ces résultats démontrent une modulation inédite de l'effet de l'antidépresseur par sa combinaison avec un inhibiteur de connexine. Cette modulation se manifeste par une potentialisation de l'effet de l'antidépresseur qui, à une dose 25 fois moins importante que la dose pharmacologique habituellement utilisée, permet d'obtenir, lorsqu'il est associé aux molécules anticonnexines, le même effet électroencéphalographique que l'antidépresseur seul (à la dose pharmacologique). Cette potentialisation de l'effet d'antidépresseur par l'anticonnexine permettrait donc de réduire les doses d'antidépresseur d'au moins 25 fois et ce avec le minimum d'effet propre de l'anticonnexine. Le bénéfice de dose mesuré de l'association Venlafaxine et anticonnexine serait donc supérieur à 25.

**Exemple 9 : Effet d'un troisième antidépresseur en combinaison avec un inhibiteur de connexines**

Afin de poursuivre l'évaluation de l'hypothèse d'un rôle modulateur global des connexines dans l'activité cérébrale, les conséquences électrophysiologiques d'un troisième traitement pharmacologique antidépresseur associé à des molécules anti-connexines ont été étudiées.

Dans ce cadre, les effets de l'escitalopram en association avec un inhibiteur des connexines, l'acide méclofénamique (MFA) ont été étudiés. L'escitalopram est un antidépresseur, indiqué dans les états dépressifs majeurs, la prévention des attaques de panique avec ou sans agoraphobie. L'escitalopram est un inhibiteur pur de la recapture de la sérotonine, de profil intermédiaire, d'efficacité comparable à celle des imipraminiques, présentant une liste considérable d'effets indésirables aux doses thérapeutiques (nausées, céphalées, insomnie, constipation, somnolence, sueurs, trouble de l'éjaculation, diarrhée, vomissement, vertiges, tremblements, paresthésies, palpitation, hypotension orthostatique, prurit).

**9.1 Effet de l'Escitalopram**

Dans un premier temps, le modèle expérimental a été évalué dans la mesure où aucune donnée bibliographique sur l'effet EEG de l'escitalopram chez le rat vigile n'est disponible ; en revanche, différentes études sur les effets de l'escitalopram notamment sur les modification EEG du sommeil fournissent les doses pharmacologiques de Escitalopram en traitement aigue qui sont généralement de 1 à 10mg/kg par voie intrapéritonéale (Sanchez C.,, Pharmacol Biochem Behav. 2007 Mar ; 86(3) :468-76)

Des enregistrements de l'effet de l'escitalopram seul ont été réalisés à deux doses, 0,8mg/kg et 4mg/kg administrée par voie intrapéritonéale (soit 1 à 5 fois inférieures à la dose habituellement utilisée en traitement aigu chez le rat).

L'analyse spectrale des EEG préfrontaux met en évidence une augmentation significative de puissance, selon la dose d'escitalopram, des composantes de 6 à 8Hz (environ 150% pour la dose 0,8mg/kg et 200% pour la dose 4mg/kg) et des composantes de 14-16Hz, et dans une moindre mesure les composantes de 22-24Hz dès la première heure et diminuant à la deuxième heure (cf. figure 10).

**9.2 Effet de l'association Escitalopram et Anticonnexine**

Dans un second temps, l'influence du système jonctionnel des connexines sur l'effet électrophysiologique de l'escitalopram a été étudiée.

Lors du traitement combinant l'antidépresseur (Escitalopram, 0,8mg/kg par voie intrapéritonéale) et l'inhibiteur de connexine (MFA, 0,4mg/kg par voie intrapéritonéale), l'analyse spectrale des EEG met en évidence, dès la première heure, une augmentation significative de puissance pour les composantes de 6 à 8Hz (environ 450%), les composantes 14-16Hz (environ 230%) et les composantes 22-24Hz (environ 140%). Cette augmentation de puissance se poursuit à la deuxième heure avec diminution d'amplitude dans les différentes composantes (majoritairement pour les composantes 8-10Hz) (cf. figure 10).

Cette augmentation significative de la synchronisation correspond parfaitement au spectre de l'escitalopram seul et correspond donc à une potentialisation considérable de l'effet de l'escitalopram par le blocage du système jonctionnel des connexines. En outre, à l'instar de la Clozapine, de la Paroxetine et du Modafinil, ce mécanisme de renforcement de l'effet de l'escitalopram par l'anticonnexine suit la même évolution temporelle que l'escitalopram seul. En effet, l'escitalopram seul ou en association avec l'anticonnexine entraîne des effets EEG qui sont maximaux à la première heure et s'estompe à la deuxième heure. Ainsi la potentialisation par l'anticonnexine de l'escitalopram conserve les propriétés d'évolution temporelles et renforce la notion d'un système de modulation par les connexines non limité à un seul système de neurotransmission.

En définitive, cette potentialisation du système Serotoninergique confirme l'hypothèse d'un rôle modulateur des activités électrophysiologiques par le système jonctionnel des connexines.

Enfin, ces résultats démontrent encore une fois la modulation de l'effet d'un antidépresseur par sa combinaison avec un inhibiteur de connexine. Cette modulation se manifeste par une potentialisation de l'effet de l'antidépresseur qui, à une dose 5 fois moins importante que la dose pharmacologique, est capable, lorsqu'il est associé à une molécule anticonnexine, de générer le même effet électroencéphalographique que l'antidépresseur seul (à la dose pharmacologique). Cette potentialisation de l'effet d'antidépresseur par l'anticonnexine permettrait donc de réduire les doses d'antidépresseur d'au moins 5 fois et ce avec le minimum d'effet propre de l'anticonnexine. Le bénéfice de dose mesuré de l'association Escitalopram et anticonnexine serait donc supérieur à 5.

## Exemple 10 : Effet d'un quatrième antidépresseur en combinaison avec un inhibiteur de connexines

Afin de poursuivre l'évaluation de l'hypothèse d'un rôle modulateur global des connexines dans l'activité cérébrale, les conséquences électrophysiologiques d'un quatrième traitement pharmacologique antidépresseur associé à des molécules anti-connexines ont été étudiées.

Dans ce cadre, les effets du Bupropion en association avec un inhibiteur des connexines, l'acide méclofénamique (MFA) ont été étudiés. Le Bupropion est un antidépresseur, indiqué pour l'aide au sevrage tabagique chez les sujets présentant une dépendance à la nicotine. Le Bupropion est un inhibiteur de la recapture de la noradrénaline et dopamine, présentant une liste considérable d'effets indésirables aux doses thérapeutiques (éruption cutanée, prurit, fièvre, nausées, céphalées, insomnie, vertiges, constipation, vomissement, ataxie,acouphène, confusion mentale, troubles visuels).

## 10.1 Effet de du Bupropion

Dans un premier temps, le modèle expérimental a été évalué dans la mesure où aucune donnée bibliographique sur l'effet EEG du Bupropion chez le rat vigile n'est disponible ; en revanche, différentes études sur les effets du Bupropion notamment sur les modifications EEG du sommeil fournissent les doses pharmacologiques de Bupropion en traitement aigue qui sont généralement de 5 à 150mg/kg par voie intrapéritonéale (Henshall DC., Neuropsychiatr Dis Trat. 2009; 5 :189-206).

Des enregistrements de l'effet du Bupropion seul ont été réalisés à trois doses, 0,16mg/kg, 0,8mg/kg et 4mg/kg administrée par voie intrapéritonéale (10 à 150 fois inférieures à la dose habituellement utilisée en traitement aigu chez le rat).

L'analyse spectrale des EEG préfrontaux met en évidence une augmentation significative de puissance, selon la dose de Bupropion, des composantes de 8 à 10Hz (environ 180% pour la dose 0,16mg/kg, 450% pour la dose 0,8mg/kg et 280% pour la dose 4mg/kg) et des composantes de 15-18Hz et dans une moindre mesure les composantes de 23-25Hz dès la première heure. A la deuxième heure, une augmentation significative par rapport à la première heure est observée pour les mêmes composantes pour les doses 0,16mg/kg et 4mg/kg alors que l'effet s'estompe pour la dose 0,8mg/kg (figure 11).

Ceci indique un effet-dose du Bupropion en « U » inversé à la première heure (comme schématisé en figure 13 pour la Sertraline) et un effet dose classique à la deuxième heure.

Ces effets complexes dans le temps suivant la dose suggèrent l'intervention de différents systèmes de neurotransmission ou groupes de neurones en fonction de la dose de Bupropion.

## 10.2. Effet de l'association Bupropion et Anticonnexine

Dans un second temps, l'influence du système jonctionnel des connexines sur l'effet électrophysiologique du Bupropion a été étudiée.

Lors du traitement combinant l'antidépresseur (Bupropion, 0,16mg/kg par voie intrapéritonéale) et l'inhibiteur de connexine (MFA, 0,4 mg/kg par voie intrapéritonéale), l'analyse spectrale des EEG met en évidence, dès la première heure, une augmentation significative de puissance pour les composantes de 8 à 10Hz (environ 400%), les composantes 15-18Hz (environ 230%) et les composantes 23-25Hz (environ 140%). Cette augmentation de puissance se poursuit à la deuxième heure avec diminution d'amplitude dans les différentes composantes (majoritairement pour les composantes 8-10Hz et 15-18Hz) (cf figure 11).

Cette augmentation significative de la synchronisation correspond au spectre du Bupropion seul à la dose de 0,8mg/kg de Bupropion seul et signifie donc à une potentialisation considérable de l'effet du Bupropion par le blocage du système jonctionnel des connexines. En outre, ce mécanisme de renforcement de l'effet du Bupropion par l'anticonnexine suit la même évolution temporelle que du Bupropion seul à une dose plus élevée, rendant compte de la complexité de l'évolution temporelle des effets du Bupropion en fonction de la dose, précédemment signalée (§10.1). En effet, le Bupropion seul à la dose de 0,8mg/kg ou à dose faible (0,16mg/kg) en association avec l'anticonnexine, entraîne des effets EEG qui sont maximaux à la première heure et s'estompe à la deuxième heure. Ainsi la potentialisation particulière du Bupropion par l'anticonnexine confirme et renforce la notion d'un système de modulation par les connexines non limité à un seul système de neurotransmission.

En définitive, cette potentialisation des systèmes noradrénergique et dopaminergique confirme l'hypothèse d'un rôle modulateur des activités électrophysiologiques par le système jonctionnel des connexines.

Enfin, ces résultats démontrent encore la modulation de l'effet d'un antidépresseur comme le Bupropion par sa combinaison avec un inhibiteur de connexine. Cette modulation se manifeste par une potentialisation de l'effet de l'antidépresseur qui, à une dose 5 fois moins importante que la dose pharmacologique, engendre les mêmes effets électroencéphalographiques qu'à la dose pharmacologique lorsqu'il est administré en association avec la molécule anticonnexine. Cette potentialisation de l'effet de l'antidépresseur par l'anticonnexine permettrait donc de réduire les doses de Bupropion d'au moins 5 fois et ce avec le minimum d'effet propre de l'anticonnexine. Le bénéfice de dose mesuré de l'association Bupropion et anticonnexine serait donc supérieur à 5.

### Exemple 11 : Effet d'un cinquième antidépresseur en combinaison avec un inhibiteur de connexines

Afin de poursuivre l'évaluation de l'hypothèse d'un rôle modulateur global des connexines dans l'activité cérébrale, les conséquences électrophysiologiques d'un cinquième traitement pharmacologique antidépresseur associé à des molécules anti-connexines ont été étudiées. Dans ce cadre, les effets de la Sertraline en association avec un inhibiteur des connexines, l'acide méclofénamique (MFA), ont été étudiés.

La Sertraline est un antidépresseur, indiqué dans les états dépressifs majeurs de l'adulte, les troubles obsessionnels compulsifs de l'adulte et l'enfant, et la prévention de récidives dépressives chez les patients présentant un trouble unipolaire. La Sertraline est un inhibiteur spécifique de la recapture de la sérotonine, présentant une liste considérable d'effets indésirables aux doses thérapeutiques (rashs cutanées, prurit, nausées, céphalées, insomnie, vertiges, vomissement, troubles extrapyramidaux, trouble de l'éjaculation, constipation, troubles visuels, tachycardie).

### 11.1 Effet de la sertraline

Dans un premier temps, le modèle expérimental a été évalué dans la mesure où aucune donnée bibliographique sur l'effet EEG de la sertraline chez le rat vigile n'est disponible ; En revanche, différentes études sur les effets de la sertraline notamment sur les modifications EEG du sommeil fournissent les doses pharmacologiques de Sertraline en traitement aigue qui sont généralement de 4 à 40mg/kg par voie intrapéritonéale (Freo U., Neurosci Lett. 2008; 436(2) : 148-52).

Des enregistrements de l'effet de la sertraline seule ont été réalisés à trois doses, 0,16mg/kg, 0,8mg/kg et 4mg/kg administrée par voie intrapéritonéale (1 à 25 fois inférieures à la dose habituellement utilisée en traitement aigu chez le rat).

L'analyse spectrale des EEG préfrontaux met en évidence une augmentation significative de puissance, selon la dose de Sertraline, des composantes de 7 à 9Hz (environ 260% pour la dose 0,16mg/kg et la dose 0,8mg/kg et 150% pour la dose 4mg/kg) et des composantes de 14-17Hz et dans une moindre mesure les composantes de 23-25Hz dès la première heure. A la deuxième heure, une augmentation significative par rapport à la première heure est observée pour les mêmes composantes pour les doses 0,8mg/kg (900%) et 4mg/kg (450%) alors que l'effet s'estompe légèrement pour la dose 0,16mg/kg (figure 12).

Ceci indique un effet-dose de la sertraline en « U » inversé majoritairement à la deuxième heure (comme schématisé en figure 13). Ces effets complexes dans le temps suivant la dose, comme pour le Bupropion, suggèrent l'intervention de différents systèmes de neurotransmission ou groupes de neurones en fonction de la dose de Sertraline.

### 11.2 Effets de l'association Bupropion et Anticonnexine

Dans un second temps, l'influence du système jonctionnel des connexines sur l'effet électrophysiologique de la sertraline a été étudiée.

Lors du traitement combinant l'antidépresseur (Sertraline, 0,16mg/kg par voie intrapéritonéale) et l'inhibiteur de connexine (MFA, 0,4mg/kg par voie intrapéritonéale), l'analyse spectrale des EEG met en évidence, dès la première heure, une augmentation significative de puissance pour les composantes de 7 à 9Hz (environ 400%), les composantes 14-17Hz (environ 300%) et les composantes 23-25Hz (environ 160%). Cette augmentation de puissance se poursuit à la deuxième heure avec une augmentation considérable d'amplitude dans les différentes composantes (majoritairement pour les composantes 7-9Hz (environ 1000%) et 14-17Hz (environ 700%) (figure 12).

Cette augmentation significative de la synchronisation à la deuxième heure correspond au spectre de la sertraline seule à la dose de 0,8mg/kg de sertraline seule et signifie donc à une potentialisation considérable de l'effet de la sertraline par le blocage du système jonctionnel des connexines. En outre, ce mécanisme de renforcement de l'effet de la sertraline par l'anticonnexine suit la même évolution temporelle que de la sertraline seule à une dose plus élevée, rendant compte de la complexité de l'évolution temporelle des effets de la sertraline en fonction de la dose, précédemment signalée (§11.1).

En effet, la sertraline seule à la dose de 0,8mg/kg ou à dose faible (0,16mg/kg) en association avec l'agent anticonnexine, entraîne des effets EEG qui sont maximaux à la deuxième heure. Ainsi la potentialisation particulière de la sertraline par l'anticonnexine confirme et renforce la notion d'un système de modulation par les connexines non limité à un seul système de neurotransmission.

En définitive, cette potentialisation du système sérotoninergique confirme l'hypothèse d'un rôle modulateur des activités électrophysiologiques par le système jonctionnel des connexines.

Enfin, ces résultats démontrent une nouvelle fois la modulation de l'effet d'un antidépresseur (la sertraline) par sa combinaison avec un inhibiteur de connexine. Cette modulation se manifeste par une potentialisation de l'effet de l'antidépresseur à une dose 5 fois moins importante que la dose pharmacologique. Cette potentialisation de l'effet de l'antidépresseur par l'anticonnexine permettrait donc de réduire les doses de sertraline d'au moins 5 fois et ce avec le minimum d'effet propre de l'anticonnexine. Le bénéfice de dose mesuré de l'association sertraline et anticonnexine serait donc supérieur à 5.

**Bibliographie**

Alldredge BT.: J Clin Pathol. May 12 2008 ;

Bai D, J Pharmacol Exp Ther, 2006 Dec ; 319(3) :1452-8

Burt JM, et al, Circ Research. 1989 ; 65 :829-37

Chaytor AT et al, J. Physiol 1997 ; 503 :99-110

Dahl G. et al, Biophys J, 1994 ; 67 :1816-22

De saint Hilaire Z., Neuroreport. 2001 Nov 16;12(16):3533-7

Dimpfel W, British Journal of Pharmacology 2007, 152, 538-548

Fabrizi GM, Brain 2007 feb, 130(Pt2):394-403

Figueroa XF, Alviña K, Martinez AD, et al. : Microvasc Res. 2004;68:247-57

Freo U., Neurosci Lett. 2008; 436(2) :148-52

Fukuda T., Neuroscientist 2007 ; 13(3)199-207

Galderisi S, Methods Find Exp Clin Pharmacol, 2002, 24, 85-89

Giepmans BN.: Cardiovasc Res. 2004;62:233-45

Guan X. et al, J. Cell Biol 1997; 139 :1785-92

Harks EG, The Journal of Pharmacology and Experimental Therapeutics 2001 Sep, 298(3) :1033-41

Harks EG, The Journal of Pharmacology and Experimental Therapeutics 2001 Sep, 298(3) :1033-41

Henshall DC., Neuropsychiatr Dis Trat. 2009; 5 :189-206

Hofer A et al, Glia 1998 ; 24 :141-54

Kale AY 2006, Brain Research Bulletin 2006, jul 15 ; 135 (1-2) :1-6

Kola I, Nat Rev Drug Discov. 2004 Aug;3(8):711-5.

Lai-Cheong JE, Arita K & McGrath JA. : J Invest Dermatol. 2007;127:2713-25,

Locke D. et al, J Biol Chem 2004 ; 279: 22883-92

Mandema & Danhof, Clin. Pharmacokinet. 1992, 23, 191-215

Meda P, Médecine/Sciences 1996; 12 :909-920

Meyer RA, J. Cell Biol. 1992 ; 119 : 179-89

Negri L, The Journal of Neuroscience, 2006 Jun 21;26(25):6716-27

Pan F, Vis Neurosciences 2007, Jul-Aug; 24(4):609-18

Postma FR, J Cell Biol 1998 Mar 9, 140(5):1199-209 ;

Robledo P., Alcohol Clin Exp Res. 1994 Apr ; 18(2)-363-8

Salameh A, Biochimica et Biophysica Acta 1719 (2005) 36-58

Salin-Pascual RJ., Psychopharmacology. 1997 Feb ; 129(3) :295-6

Sanchez C., Pharmacol Biochem Behav. 2007 Mar;86(3):468-76

Scemes E, Glia 2008 Jan 15,56(2): 145-53 ;

Sebban C., British Journal of Pharmacology (1999

Shaw RM, Cell 2007, February 9, 128(3):547-60;

Srinivas M, Molecular Pharmacology 2003 jun, 63(6) :1389-97
Srinivas M, PNAS 2001, 98 :10942-10947 ;
Tejwani GA, Brain Res. 1998 Jun 29;797(2):305-12
Wellershaus K, Exp Cell Res. 2008
Winneker RC, Steroids. 2003 Nov;68(10-13):915-20
Yao J, Hiramatsu N, Zhu Y, et al. : J Am Soc Nephrol. 2005;16:58-67 ;
Yao J, Morioka T & Oite T.: Kidney Int. 2000;57:1915-26.

**Revendications**

1. Produit contenant au moins un agent bloquant les connexines et une drogue psychotrope, comme produits de combinaison pour une utilisation simultanée, séparée ou étalée dans le temps, chez des patients souffrant de troubles psychiatriques et/ou neurodégénératifs.

2. Produit selon la revendication 1, **caractérisé en ce que** l'agent bloquant les connexines est choisi dans le groupe comprenant l'acide méclofénamique, l'acide 18-β-glycyrrhétinique, la méfloquine et le 2-APB, et est préférentiellement l'acide méclofénamique.

3. Produit selon l'une des revendications 1 et 2, **caractérisé en ce que** la drogue psychotrope est un effecteur dopaminergique, GABAergique, adrénergique, acétylcholinergique, sérotoninergique, opioïdergique, adénosinergique, ionotropique, histaminergique, IMAO, Catechol-O-méthyl transférase; DOPA decarboxylase, noradrenergique:

4. Produit selon la revendication 3, **caractérisé en ce que** la drogue psychotrope est un effecteur dopaminergique choisi parmi la loxapine, l'acepromazine, le méthylphenidate, l'amantadine, le pergolide, la lisuride, la bromocriptine, le ropinirole, l'apomorphine, l'aripiprazole, le sulpiride, l'amisulpride, le sultopride, le tiapride, le pimozide, la risperidone, l'haloperidol, le penfluridol, le zuclopenthixol, le bupropion.

5. Produit selon la revendication 3, **caractérisé en ce que** la drogue psychotrope est un effecteur GABAergique choisi parmi le tiagabine, le topiramate, le clorazepate, le diazepam, le clonazepam, l'oxazepam, le lorazepam, le bromazepam, le lormetazepam, le nitrazepam, le clotiazepam, l'alprozolam, l'estazolam, le triazolam, le loprazolam, l'etifoxin, le meprobamate, le zopiclone, le zolpidem, le phenobarbital, le felbamate, la vigabatrine.

6. Produit selon la revendication 3, **caractérisé en ce que** la drogue psychotrope est un effecteur adrénergique choisi parmi la dihydroergotamine, le modafinil, l'adrafinil, la mirtazapine, l'oxetorone.

7. Produit selon la revendication 3, **caractérisé en ce que** la drogue psychotrope est un effecteur acétylcholinergique choisi parmi la sulbutiamine, la tropatepine, le trihexyphenidyle.

8. Produit selon la revendication 3, **caractérisé en ce que** la drogue psychotrope est un effecteur sérotinergique choisi parmi la chlorpromazine, la trimipramine, la clozapine, l'olanzapine, la cyamemazine, le flupentixol, le nefopam, la fluvoxamine, la clomipramine, la sertraline, la fluoxetine, le citalopram, l'escitalopram, la paroxetine, l'amitriptyline, la duloxetine, la venlafaxine, la buspirone, la carpipramine, le zolmitriptan, le sumatriptan, le naratriptan, l'indoramine, l'ergotamine, le tartrate d'ergotamine, le pizotifène, la pipamperone, le methysergide, la pizotyline, la tianeptine, le milnacipran, l'amitriptyline, la trimipramine, la viloxazine, la tianeptine, le millepertuis, le lithium.

9. Produit selon la revendication 3, **caractérisé en ce que** la drogue psychotrope est un effecteur opioïdergique choisi parmi la nalbuphine, la buprenorphine, la pethidine, la codeine, le tramadol, la morphine, l'hydromorphone, l'oxycodone, la methadone, le dextroproxyphène, le meperidine, le fentanyl, le naltrexone, le chlorhydrate de morphine.

10. Produit selon la revendication 3, **caractérisé en ce que** la drogue psychotrope est un effecteur adénosinergique choisi parmi la carbamazepine, l'oxcarbazepine.

11. Produit selon la revendication 3, **caractérisé en ce que** la drogue psychotrope est un effecteur ionotropique choisi parmi la flunarizine, l'ethosuximide, le levetiracetam, la lamotrigine, la fosphenytoine, la phenytoine.

12. Produit selon la revendication 3, **caractérisé en ce que** la drogue psychotrope est un effecteur histaminergique choisi parmi la niaprazine, l'hydroxyzine et la doxylamine, ou un effecteur Monoamine oxydase choisi parmi le moclobemide, la selegiline et l'iproniazid, ou un effecteur Catéchol-O-méthyl transférase choisi parmi l'entacapone, et le tolcapone, ou un effecteur DOPA decarboxylase choisi parmi le benserazide et la carbidopa, ou un effecteur noradrenergique choisi parmi la miansérine, la désipramine, le moclobémide, et le bupropion, ou un effecteur agissant au niveau du système limbique choisi parmi la gabapentine et la captodiamine.

**13.** Produit selon la revendication 3, **caractérisé en ce que** la drogue psychotrope est choisie dans le groupe comprenant la clozapine, le modafinil, la paroxétine, le diazepam l'escitalopram, la sertraline, la venlafaxine et le bupropion.

**14.** Utilisation d'au moins un agent bloquant les connexines pour préparer un médicament destiné à être administré avant, en même temps ou après une drogue psychotrope, en particulier telle que définie à l'une des revendications 1 à 13, pour traiter un patient souffrant de troubles psychiatriques et/ou neurodégénératifs.

**15.** Utilisation d'au moins un agent bloquant les connexines selon la revendication 14, pour potentialiser l'effet d'une drogue psychotrope chez des patients souffrant de troubles psychiatriques et/ou neurodégénératifs.

**16.** Utilisation d'au moins un agent bloquant les connexines selon les revendications 14 ou 15, pour diminuer les doses de ladite drogue psychotrope et/ou limiter les effets secondaires de ladite drogue psychotrope, et/ou réduire les effets d'échappement et de sevrage.

**Claims**

**1.** Product containing at least one connexin-blocking agent and a psychotropic drug, as combination products for simultaneous, separate or sequential use over time, in patients suffering from psychiatric and/or neurodegenerative disorders.

**2.** Product according to claim 1, **characterized in that** the connexin-blocking agent is chosen from the group including meclofenamic acid, 18-β-glycyrrhetinic acid, mefloquine and 2-APB, and is preferably meclofenamic acid.

**3.** Product according to claims 1 and 2, **characterized in that** the psychotropic drug is a dopaminergic, GABAergic, adrenergic, acetylcholinergic, serotoninergic, opioidergic, adenosinergic, ionotropic, histaminergic, IMAO, Catechol-O-methyl transferase, DOPA decarboxylase or noradrenergic effector.

**4.** Product according to claim 3, **characterized in that** the psychotropic drug is a dopaminergic effector chosen from loxapine, acepromazine, methylphenidate, amantadine, pergolide, lisuride, bromocriptine, ropinirole, apomorphine, aripiprazole, sulpiride, amisulpride, sultopride, tiapride, pimozide, risperidone, haloperidol, penfluridol, zuclopenthixol or bupropion.

**5.** Product according to claim 3, **characterized in that** the psychotropic drug is a GABAergic effector chosen from tiagabine, topiramate, clorazepate, diazepam, clonazepam, oxazepam, lorazepam, bromazepam, lormetazepam, nitrazepam, clotiazepam, alprozolam, estazolam, triazolam, loprazolam, etifoxin, meprobamate, zopiclone, zolpidem, phenobarbital, felbamate or vigabatrine.

**6.** Product according to claim 3, **characterized in that** the psychotropic drug is an adrenergic effector chosen from dihydroergotamine, modafinil, adrafinil, mirtazapine or oxetorone.

**7.** Product according to claim 3, **characterized in that** the psychotropic drug is an acetylcholinergic effector chosen from sulbutiamine, tropatepin or trihexyphenidyl.

**8.** Product according to claim 3, **characterized in that** the psychotropic drug is a serotoninergic effector chosen from chlorpromazine, trimipramine, clozapine, olanzapine, cyamemazine, flupentixol, nefopam, fluvoxamine, clomipramine, sertraline, fluoxetine, citalopram, escitalopram, paroxetine, amitriptyline, duloxetine, venlafaxine, buspirone, carpipramine, zolmitriptan, sumatriptan, naratriptan, indoramine, ergotamine, ergotamine tartrate, pizotifene, pipamperone, methysergide, pizotyline, tianeptine, milnacipran, amitriptyline, trimipramine, viloxazine, tianeptine, hypericum or lithium.

**9.** Product according to claim 3, **characterized in that** the psychotropic drug is an opioidergic effector chosen from nalbuphine, buprenorphine, pethidine, codeine, tramadol, morphine, hydromorphone, oxycodone, methadone, dextropropoxyphene, meperidine, fentanyl, naltrexone or morphine hydrochloride.

10. Product according to claim 3, **characterized in that** the psychotropic drug is an adenosinergic effector chosen from carbamazepine or oxcarbazepine.

11. Product according to claim 3, **characterized in that** the psychotropic drug is an ionotropic effector chosen from flunarizine, ethosuximide, levetiracetam, lamotrigine, fosphenytoin or phenytoin.

12. Product according to claim 3, **characterized in that** the psychotropic drug is a histaminergic effector chosen from niaprazine, hydroxyzine or doxylamine, or a monoamine oxidase effector chosen from moclobemide, selegiline or iproniazid, or a catechol-O-methyl transferase effector chosen from entacapone or tolcapone, or a DOPA decarboxylase effector chosen from benserazide or carbidopa, or a noradrenergic effector chosen from mianserine, desipramine, moclobemide or bupropion, or an effector acting on the limbic system, chosen from gabapentin or captodiamine.

13. Product according to claim 3, **characterized in that** the psychotropic drug is chosen from the group including clozapine, modafinil, paroxetine, diazepam, escitalopram, sertraline, venlafaxine and bupropion.

14. Use of at least one connexin-blocking agent to prepare a drug intended to be administered before, simultaneously to or after a psychotropic drug, in particular as defined in one of claims 1 to 13, to treat a patient suffering from psychiatric and/or neurodegenerative disorders.

15. Use of at least one connexin-blocking agent according to claim 14, to potentiate the effect of a psychotropic drug in patients suffering from psychiatric and/or neurodegenerative disorders.

16. Use of at least one connexin-blocking agent according to claims 14 or 15, to reduce the doses of said psychotropic drug and/or to limit the adverse effects of said psychotropic drug, and/or to reduce the effects of failure and withdrawal.

**Patentansprüche**

1. Erzeugnis, welches mindestens ein Mittel, welches die Connexine blockiert, und ein psychotropes Arzneimittel enthält, als Kombinationsprodukte für eine gleichzeitige, getrennte oder zeitlich gestaffelte Verwendung bei Patienten, die unter psychiatrischen und/oder neurodegenerativen Störungen leiden.

2. Erzeugnis nach Anspruch 1, **dadurch gekennzeichnet, dass** das Mittel, welches die Connexine blockiert, in der Gruppe, welche Meclofenaminsäure, 18-β-Glycyrrhetinsäure, Mefloquin und 2-APB umfasst, ausgewählt ist und vorzugsweise Meclofenaminsäure ist.

3. Erzeugnis nach einem der Ansprüche 1 und 2, **dadurch gekennzeichnet, dass** das psychotrope Arzneimittel ein dopaminerger, GABAerger, adrenerger, acetylcholinerger, serotonerger, opioiderger, adenosinerger, ionotropischer, histaminerger, IMAO-, Catechol-O-methyltransferase-, DOPA-Decarboxylase-, noradrenerger Effektor ist.

4. Erzeugnis nach Anspruch 3, **dadurch gekennzeichnet, dass** das psychotrope Arzneimittel ein dopaminerger Effektor, der unter Loxapin, Acepromazin, Methylphenidat, Amantadin, Pergolid, Lisurid, Bromcriptin, Ropinirol, Apomorphin, Aripiprazol, Sulpirid, Amisulprid, Sultoprid, Tiaprid, Pimozid, Risperidon, Haloperidol, Penfluridol, Zuclopenthixol, Bupropion ausgewählt ist, ist.

5. Erzeugnis nach Anspruch 3, **dadurch gekennzeichnet, dass** das psychotrope Arzneimittel ein GABAerger Effektor, der unter Tiagabin, Topiramat, Clorazepat, Diazepam, Clonazepam, Oxazepam, Lorazepam, Bromazepam, Lormetazepam, Nitrazepam, Clotiazepam, Alprozolam, Estazolam, Triazolam, Loprazolam, Etifoxin, Meprobamat, Zopiclon, Zolpidem, Phenobarbital, Felbamat, Vigabatrin ausgewählt ist, ist.

6. Erzeugnis nach Anspruch 3, **dadurch gekennzeichnet, dass** das psychotrope Arzneimittel ein adrenerger Effektor, der unter Dihydroergotamin, Modafinil, Adrafinil, Mirtazapin, Oxetoron ausgewählt ist, ist.

7. Erzeugnis nach Anspruch 3, **dadurch gekennzeichnet, dass** das psychotrope Arzneimittel ein acetylcholinerger Effektor, der unter Sulbutiamin, Tropatepin, Trihexyphenidyl ausgewählt ist, ist.

8. Erzeugnis nach Anspruch 3, **dadurch gekennzeichnet, dass** das psychotrope Arzneimittel ein serotonerger Effektor, der unter Chlorpromazin, Trimipramin, Clozapin, Olanzapin, Cyamemazin, Flupentixol, Nefopam, Fluvoxamin, Clomipramin, Sertralin, Fluoxetin, Citalopram, Escitalopram, Paroxetin, Amitriptylin, Duloxetin, Venlafaxin, Buspiron, Carpipramin, Zolmitriptan, Sumatriptan, Naratriptan, Indoramin, Ergotamin, Ergotamintartrat, Pizotifen, Pipamperon, Methysergid, Pizotylin, Tianeptin, Milnacipran, Amitriptylin, Trimipramin, Viloxazin, Tianeptin, Johanniskraut, Lithium ausgewählt ist, ist.

9. Erzeugnis nach Anspruch 3, **dadurch gekennzeichnet, dass** das psychotrope Arzneimittel ein opioiderger Effektor, der unter Nalbuphin, Buprenorphin, Pethidin, Codein, Tramadol, Morphin, Hydromorphon, Oxycodon, Methadon, Dextroproxyphen, Meperidin, Fentanyl, Naltrexon, Morphinhydrochlorid ausgewählt ist, ist.

10. Erzeugnis nach Anspruch 3, **dadurch gekennzeichnet, dass** das psychotrope Arzneimittel ein adenosinerger Effektor, der unter Carbamazepin, Oxcarbazepin ausgewählt ist, ist.

11. Erzeugnis nach Anspruch 3, **dadurch gekennzeichnet, dass** das psychotrope Arzneimittel ein ionotropischer Effektor, der unter Flunarizin, Ethosuximid, Levetiracetam, Lamotrigin, Fosphenytoin, Phenytoin ausgewählt ist, ist.

12. Erzeugnis nach Anspruch 3, **dadurch gekennzeichnet, dass** das psychotrope Arzneimittel ein histaminerger Effektor, der unter Niaprazin, Hydroxyzin und Doxylamin ausgewählt ist, oder ein Monoaminoxydase-Effektor, der unter Moclobemid, Selegilin und Iproniazid ausgewählt ist, oder ein Catechol-O-methyltransferase-Effektor, der unter Entacapon und Tolcapon ausgewählt ist, oder ein DOPA-Decarboxylase-Effektor, der unter Benserazid und Carbidopa ausgewählt ist, oder ein noradrenerger Effektor, der unter Mianserin, Desipramin, Moclobemid und Bupropion ausgewählt ist, oder ein Effektor, der seine Wirkung auf der Ebene des limbischen Systems entfaltet, der unter Gabapentin und Captodiamin ausgewählt ist, ist.

13. Erzeugnis nach Anspruch 3, **dadurch gekennzeichnet, dass** das psychotrope Arzneimittel in der Gruppe, welche Clozapin, Modafinil, Paroxetin, Diazepam, Escitalopram, Sertralin, Venlafaxin und Bupropion umfasst, ausgewählt wird.

14. Verwendung von mindestens einem Mittel, welches die Connexine blockiert, zum Herstellen eines Arzneimittels, welches dazu bestimmt ist, vor, zur gleichen Zeit wie oder nach ein(em) psychotropes/n Arzneimittel verabreicht zu werden, insbesondere wie in einem der Ansprüche 1 bis 13 definiert, um einen Patienten, der unter psychiatrischen und/oder neurodegenerativen Störungen leidet, zu behandeln.

15. Verwendung von mindestens einem Mittel, welches die Connexine blockiert, nach Anspruch 14 zum Potenzieren der Wirkung eines psychotropen Arzneimittels bei Patienten, die unter psychiatrischen und/oder neurodegenerativen Störungen leiden.

16. Verwendung von mindestens einem Mittel, welches die Connexine blockiert, nach den Ansprüchen 14 oder 15, um die Dosen des psychotropen Arzneimittels zu verringern und/oder die Nebenwirkungen des psychotropen Arzneimittels zu begrenzen und/oder die Escape- bzw. Wirkungsverlusts- und Entzugs- bzw. Entwöhnungswirkungen zu verringern.

Figure 1

# Figure 2

**1ère Heure**

**2ème Heure**

Figure 3

clozapine

Association
clozapine et anticonnexine

Anti-connexine

Figure 4

Pt/Pc                          Heure 1

-X- Anticonnexine IP
-☐-Anticonnexine IC

Pt/Pc                          Heure 2

Figure 5

Figure 6

Figure 7

**Pt/Pc**

Heure 1

—△— Anticonnexine (0,4mg/kg)

—⊖— Modafinil (125 mg/kg)

Association
Modafinil+Anticonnexine

- - - • - - - Modafinil (250 mg/kg)

**Pt/Pc**

Heure 2

Figure 8

**Pt/Pc**

Heure 1

Diazepam (1mg/kg)

Association Diazepam (1mg/kg)
+Anticonnexine (0,4mg/kg)

$\nu$

**Pt/Pc**

Heure 2

$\nu$

Figure 9

Figure 10

Figure 11

Figure 12

Figure 13

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **KOLA I.** *Nat Rev Drug Discov.,* Août 2004, vol. 3 (8), 711-5
- **NEGRI L.** *The Journal of Neuroscience,* 21 Juin 2006, vol. 26 (25), 6716-27
- **KALE AY.** *Brain Research Bulletin,* 15 Juillet 2006, vol. 135 (1-2), 1-6
- **WINNEKER RC.** *Steroids,* Novembre 2003, vol. 68 (10-13), 915-20
- **TEJWANI GA.** *Brain Res.,* 29 Juin 1998, vol. 797 (2), 305-12
- **MEDA P.** *Médecine/Sciences,* 1996, vol. 12, 909-920
- **WELLERSHAUS K.** *Exp Cell Res.,* 2008
- **GALDERISI S.** *Methods Find Exp Clin Pharmacol,* 2002, vol. 24, 85-89
- Animal models in psychopharmacology. Birkhauser Verlag, 1991
- **LECADET J ; VIDAL P ; BARIS B et al.** Médicaments psychotropes: consommation et pratiques de prescription en France métropolitaine. I. Données nationales, 2000. *Revue Médicale de l'Assurance Maladie,* Avril 2003, vol. 34 (2
- **HARKS EG.** *The Journal of Pharmacology and Experimental Therapeutics,* Septembre 2001, vol. 298 (3), 1 033-41
- **PAN F.** *Vis Neurosciences,* Juillet 2007, vol. 24 (4), 609-18
- **SRINIVAS M.** *PNAS,* 2001, vol. 98, 10942-10947
- **BURT JM et al.** *Circ Research.,* 1989, vol. 65, 829-37
- **GUAN X. et al.** *J. Cell Biol,* 1997, vol. 139, 1785-92
- **LOCKE D. et al.** *J Biol Chem,* 2004, vol. 279, 22883-92
- **BAI D.** *J Pharmacol Exp Ther,* Décembre 2006, vol. 319 (3), 1452-8
- **HOFER A et al.** *Glia,* 1998, vol. 24, 141-54
- **MEYER RA.** *J. Cell Biol.,* 1992, vol. 119, 179-89
- **DAHL G. et al.** *Biophys J,* 1994, vol. 67, 1816-22
- **CHAYTOR AT et al.** *J. Physiol,* 1997, vol. 503, 99-110
- **SRIVINAS M.** Connexins : a guide. Humana Press, 2009, 207-224
- **SRINIVAS M.** *Molecular Pharmacology,* Juin 2003, vol. 63 (6), 1389-97
- **HARKS EG.** *The Journal of Pharmacology and Experimental Therapeutics,* Septembre 2001, vol. 298 (3), 1033-41
- **SALAMEH A.** *Biochimica et Biophysica Acta,* 2005, vol. 1719, 36-58
- **YAO J ; MORIOKA T ; OITE T.** *Kidney Int.,* 2000, vol. 57, 1915-26
- **YAO J ; HIRAMATSU N ; ZHU Y et al.** *J Am Soc Nephrol.,* 2005, vol. 16, 58-67
- **FIGUEROA XF ; ALVIÑA K ; MARTINEZ AD et al.** *Microvasc Res.,* 2004, vol. 68, 247-57
- **ALLDREDGE BT.** *J Clin Pathol.,* 12 Mai 2008
- **LAI-CHEONG JE ; ARITA K ; MCGRATH JA.** *J Invest Dermatol.,* 2007, vol. 127, 2713-25
- **GIEPMANS BN.** *Cardiovasc Res.,* 2004, vol. 62, 233-45
- **SCEMES E.** *Glia,* 15 Janvier 2008, vol. 56 (2), 145-53
- **POSTMA FR.** *J Cell Biol,* 09 Mars 1998, vol. 140 (5), 1199-209
- **SHAW RM.** *Cell,* 09 Février 2007, vol. 128 (3), 547-60
- **FABRIZI GM.** *Brain,* Février 2007, vol. 130, 394-403
- **DIMPFEL W.** *Neuropsychobiology,* vol. 15 (2), 101-8
- **DIMPFEL W.** *British Journal of Pharmacology,* 2007, vol. 152, 538-548
- **MANDEMA ; DANHOF.** *Clin. Pharmacokinet.,* 1992, vol. 23, 191-215
- **PARKER TJ.** *British Journal of Pharmacology,* 2001, vol. 132, 151-158
- EEG : Bases neurophysiologiques, principes d'interprétation et de prescription. Abrégé Masson
- **GALDERISI S.** *Methods Find Exp Clin Pharmaco,* 2002, vol. 24, 85-89
- **FUKUDA T.** *Neuroscientist,* 2007, vol. 13 (3), 199-207
- **SANCHEZ C.** *Pharmacol Biochem Behav.,* Mars 2007, vol. 86 (3), 468-76
- **SEBBAN C.** *British Journal of Pharmacology,* 1999, vol. 128, 1045-1054
- **DE SAINT HILAIRE Z.** *Neuroreport,* 16 Novembre 2001, vol. 12 (16), 3533-7
- **ROBLEDO P.** *Alcohol Clin Exp Res.,* Avril 1994, vol. 18 (2), 363-8

- **SALIN-PASCUAL RJ.** *Psychopharmacology,* Février 1997, vol. 129 (3), 295-6
- **HENSHALL DC.** *Neuropsychiatr Dis Trat.,* 2009, vol. 5, 189-206
- **FREO U.** *Neurosci Lett.,* 2008, vol. 436 (2), 148-52
- **ALLDREDGE BT.** *J Clin Pathol.,* 12 Mai 2008
- **DE SAINT HILAIRE Z.** *Neuroreport.,* 16 Novembre 2001, vol. 12 (16), 3533-7
- **GUAN X. et al.** *J. Cell Biol,* vol. 139, 1785-92
- **SALAMEH A.** *Biochimica et Biophysica Acta,* vol. 1719 (2005), 36-58
- **SANCHEZ C.** *Pharmacol Biochem Behav.,* Mars 2007, vol. 86 (3), 468-76
- **SEBBAN C.** *British Journal of Pharmacology,* 1999
- **YAO J ; MORIOKA T ; OITE T.** *Kidney Int.,* 2000, vol. 57, 1915-26